# EUROPÄISCHE PATENTSCHRIFT

(11) **EP 1 651 618 B1**
(45) Veröffentlichungstag und Bekanntmachung des Hinweises auf die Patenterteilung: **03.01.2007**
(21) Anmeldenummer: 04741053.5
(22) Anmeldetag: 15.07.2004
(51) Int. Cl.: C07D 239/42

(54) **2-SUBSTITUIERTE PYRIMIDINE**
2-SUBSTITUTED PYRIMIDINES
PYRIMIDINES SUBSTITUEES EN 2

(30) Priorität: 24.07.2003 DE 10333857; 09.12.2003 DE 10357714
(43) Veröffentlichungstag der Anmeldung: 03.05.2006
(73) Patentinhaber: BASF Aktiengesellschaft, 67056 Ludwigshafen (DE)
(72) Erfinder: SCHWÖGLER, Anja, 68165 Mannheim (DE); GEWEHR, Markus, 56288 Kastellaun (DE); MÜLLER, Bernd, 67227 Frankenthal (DE); GROTE, Thomas, 67157 Wachenheim (DE); GRAMMENOS, Wassilios, 67071 Ludwigshafen (DE); TORMO I BLASCO, Jordi, 69514 Laudenbach (DE); GYPSER, Andreas, 68159 Mannheim (DE); RHEINHEIMER, Joachim, 67063 Ludwigshafen (DE); BLETTNER, Carsten, 68165 Mannheim (DE); SCHÄFER, Peter, 67308 Ottersheim (DE); SCHIEWECK, Frank, 67258 Hessheim (DE); WAGNER, Oliver, 67433 Neustadt (DE); STIERL, Reinhard, 67251 Freinsheim (DE); SCHÖFL, Ulrich, 68782 Brühl (DE); STRATHMANN, Siegfried, 67117 Limburgerhof (DE); SCHERER, Maria, 76829 Goldramstein (DE)
(86) Internationale Anmeldenummer: PCT/EP2004/007877
(87) Internationale Veröffentlichungsnummer: WO 2005/012261

(56) Entgegenhaltungen:
- WO-A-02/074753
- WO-A-03/043993

## Beschreibung

Die Erfindung betrifft 2-substituierte Pyrimidine der Formel I,

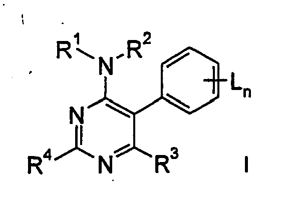

in der der Index und die Substituenten die folgende Bedeutung haben:
- n: eine ganze Zahl von 1 bis 5;
- L: Halogen, Cyano, Cyanato (OCN), C₁-C₈-Alkyl, C₂-C₈-Alkenyl, C₂-C₈-Alkinyl, C₁-C₆-Alkoxy, C₂-C₈-Alkenyloxy, C₂-C₈-Alkinyloxy, C₃-C₆-Cycloalkyl, C₄-C₆-Cycloalkenyl, C₃-C₆-Cycloalkyloxy, C₄-C₆-Cycloalkenyloxy, Nitro, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A,
- m: 0, 1 oder 2;
- A, A', A": unabhängig voneinander Wasserstoff, C₁-C₆-Alkyl, C₂-C₆-Alkenyl, C₂-C₆-Alkinyl, C₃-C₈-Cycloalkyl, C₃-C₈-Cycloalkenyl, Phenyl, wobei die organischen Reste partiell oder vollständig halogeniert sein können oder durch Nitro, Cyanato, Cyano oder C₁-C₄-Alkoxy substituiert sein können; oder A und A' zusammen mit den Atomen an die sie gebunden sind für einen fünf- bis sechsgliedrigen gesättigten, partiell ungesättigten oder aromatischen Heterocyclus, enthaltend ein bis vier Heteroatome aus der Gruppe O, N oder S, stehen;
wobei die aliphatischen Gruppen der Restedefinitionen von L ihrerseits partiell oder vollständig halogeniert sein oder eine bis vier Gruppen R^{u} tragen können:
- R^{u}: Cyano, C₁-C₆-Alkoxy, C₃-C₆-Cycloalkyl, C₂-C₈-Alkonyloxy, C₂-C₈-Alkinyloxy, C₄-C₆-Cycloalkenyl, C₃-C₆-Cycloalkyloxy, C₄-C₆-Cycloalkenyloxy -C(=O)-A, -C(=O)-O-A, -C(=O)-N(A')A, C(A')(=N-OA), N(A')A, N(A')-C(=O)-A, N(A'')-C(=O)-N(A')A, S(=O)ₘ-A, S(=O)ₘ-O-A oder S(=O)ₘ-N(A')A;
- R¹, R²: unabhängig voneinander C₁-C₆-Alkyl, C₂-C₆-Alkenyl, C₂-C₆-Alkinyl, C₃-C₆-Cycloalkyl, C₃-C₆-Halogencycloalkyl, wobei die aliphatischen Grup-pen der Restedefinitionen von R¹ und R² ihrerseits partiell oder vollständig halogeniert sein oder eine bis vier Gruppen R^{v} tragen können:
R^{v} Cyano, C₃-C₆-Cycloalkyl, C₄-C₆-Cycloalkenyl, Hydroxy, C₁-C₆-Alkoxy, C₂-C₈-Alkenyloxy, C₂-C₈-Alkinyloxy, C₃-C₆-Cycloalkyloxy, C₄-C₆-Cycloalkenyloxy, C₁-C₆-Alkylthio, -C(=O)-A, -C(=O)-O-A, -C(=O)-N(A')A, C(A')(=N-OA), N(A')A, N(A')-C(=O)-A, N(A'')-C(=O)-N(A')A, S(=O)ₘ-A, S(=O)ₘ-O-A oder S(=O)ₘ-N(A')A oder Phenyl, wobei der Phenylteil ein bis drei Reste ausgewählt aus der Gruppe: Halogen, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy, Cyano, Nitro, -C(=O)-A, -C(=O)-O-A, -C(=O)-N(A')A, C(A')(=N-OA), N(A')A tragen kann;
- R²: kann zusätzlich Wasserstoff bedeuten;
- R¹ und R²: können auch zusammen mit dem Stickstoffatom, an das sie gebunden sind, einen gesättigten oder ungesättigten fünf- oder sechsgliedrigen Ring bilden, der durch eine Ether -(-O-), Carbonyl -(C=O)-, Thio -(-S-), Sulfoxyl -(-S[=O]-) oder Sulfenyl -(-SO₂-) oder eine weitere Amino -(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
- R³: Halogen, Cyano, C₁-C₄-Alkyl, C₂-C₄-Alkenyl, C₂-C₄-Alkinyl, C₃-C₆-Cycloalkyl, C₁-C₄-Alkoxy, C₃-C₄-Alkenyloxy, C₃-C₄-Alkinyloxy, C₁-C₆-Alkylthio, Di-(C₁-C₆-alkyl)amino oder C₁-C₆-Alkylamino, wobei die Alkyl, Alkenyl und Alkinylreste von R³ durch Halogen, Cyano, Nitro, C₁-C₂-Alkoxy oder C₁-C₄-Alkoxycarbonyl substituiert sein können;
- R⁴: einer der Formeln 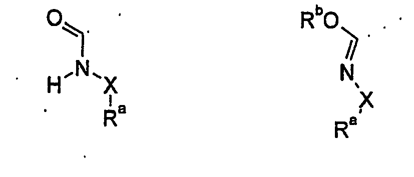 entspricht, in denen
X eine direkte Bindung, -(C=O)-, -(C=O)-NH-, -(C=O)-O-, -O-, -NR^{c}-, -CH₂-O- (C=O)-, -C=C-(C=O)-, wobei das jeweils linke Atom des Brückenglieds an das Stickstoffatom gebunden ist;
R^{a} Wasserstoff, C₁-C₆-Alkyl, C₂-C₈-Alkenyl, C₂-C₈-Alkinyl oder Benzyl;
R^{b} Wasserstoff, C₁-C₆-Alkyl, C₂-C₆-Alkenyl oder C₂-C₆-Alkinyl;
R^{c} Wasserstoff, C₁-C₆-Alkyl, C₂-C₆-Alkenyl, C₂-C₆-Alkinyl, Benzyl oder C₁-C₆-Acyl
bedeuten,
wobei die aliphatischen, alicyclischen oder aromatischen Gruppen der Restedefinitionen von R^{a}, R^{b} und/oder R^{c} ihrerseits eine bis vier Gruppen R^{w} tragen können:
R^{w} Halogen, Cyano, OR^{x}, NHR^{x}, SR^{x}, C₁-C₆-Alkyl, C₂-C₆-Alkenyl, C₂-C₈-Alkinyl, C₁-C₆-Alkoxy, C₁-C₄-Alkoxycarbonyl, C₁-C₄-Acylamino, [1,3]Dioxolane-C₁-C₄-alkyl, [1,3]Dioxane-C₁-C₄-alkyl, wobei
R^{x} Wasserstoff, C₁-C₆-Alkyl, C₂-C₆-Alkenyl, C₂-C₈-Alkinyl oder Benzyl bedeutet.

Außerdem betrifft die Erfindung ein Verfahren zur Herstellung dieser Verbindungen, 2-Pyrimidine enthaltende Mittel sowie deren Verwendung zur Bekämpfung pflanzenpathogener Schadpilze.

Aus WO-A 01/96314 sind fungizide Pyrimidine, die in 2-Stellung einen Cyanaminosubstituenten tragen, bekannt. Weiterhin sind aus WO-A 03/43993 fungizide 2-Pyrimidyl-N-methoxyamidine bekannt.

Die Wirkung der o.g. Pyrimidine ist jedoch in vielen Fällen nicht zufriedenstellend. Daher lag als Aufgabe zugrunde, Verbindungen mit verbesserter Wirksamkeit zu finden.

Demgemäß wurden die eingangs definierten Pyrimidine der Formel I gefunden. Außerdem wurden Verfahren zu ihrer Herstellung sowie sie enthaltende Mittel zur Bekämpfung von Schadpilzen gefunden.

Die Verbindungen I können auf verschiedenen Wegen erhalten werden.
1) Beispielsweise kann von den Sulfonen der Formel II ausgegangen werden, deren Herstellung in WO-A 02/074753 oder DE 10156279.9 detailliert beschrieben ist. Durch Umsetzung der Sulfone II mit Metallcyaniden III (Me⁺CN⁻) werden die Nitrile IV gewonnen. Unter Metallcyaniden sind in erster Linie Alkali- oder Erdalkalicyanide oder auch kovalente Cyanide wie Zinntetracyanid zu verstehen. 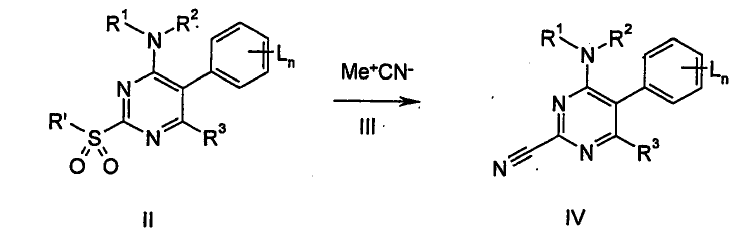
   Der Austausch der Sulfonatgruppe gegen die Nitrilgruppe erfolgt nach literaturbekannten Methoden wie sie beispielsweise in WO-A 03/043993 beschrieben sind.

Die weitere Synthese kann wie in Schema 1 dargestellt erfolgen:

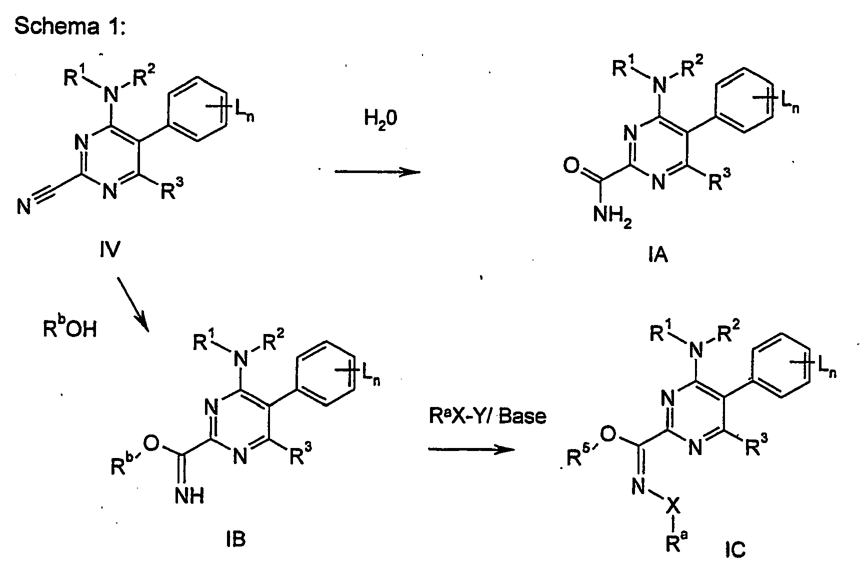

Die Nitrilverbindung IV kann unter sauren oder vorzugsweise basischen Bedingungen zum Amid IA hydrolysiert werden. Die Hydrolyse erfolgt beispielsweise unter den von Katritzky et al. in Synthesis 1989, S. 949-950 beschriebenen Bedingungen (Wasserstoffperoxid, Base, polares aprotisches Lösungsmittel). In Comprehensive Organic Chemistry, Vol 2, Sutherland, I.O. Pergamon Press, Oxford, 1979, S. 964 sind Hydrolysen von Nitrilen zu Amiden unter sauren Bedingungen beschrieben.

Alternativ hierzu kann das Pinneraddukt, das sich durch Anlagerung von in der Regel Salzsäure an das Nitril IV bildet, mit einem Alkohol der Formel R^{b}OH, wobei R^{b} die zuvor genannte Bedeutung besitzt, zum Iminoether der Formel IB umgesetzt werden. Die Alkylierung mit R^{a}X-Y, wobei R^{a} und das Brückenglied X die eingangs erwähnte Bedeutung hat und y für eine Abgangsgruppe wie Halogenid, Sulfat oder Sulfonat steht, liefert Verbindungen des Typs IC.

Die Alkylierung mit R^{a}-Y kann ausgehend von Verbindung IB oder dem Nitril IV auch mit Meerwein Salzen der Formel (R^{a})₃OBF₄ analog den in Synth. Commun., 1983, 13, S. 753 oder Helv. Chim. Acta, 1986, 69, S. 1224 aufgeführten Vorschriften durchgeführt werden. Man gelangt zu Verbindungen I, wobei X für eine direkte Bindung steht.

Eine alternative Synthese der erfindungsgemäßen Verbindungen IA ist in Schema 2 aufgeführt.

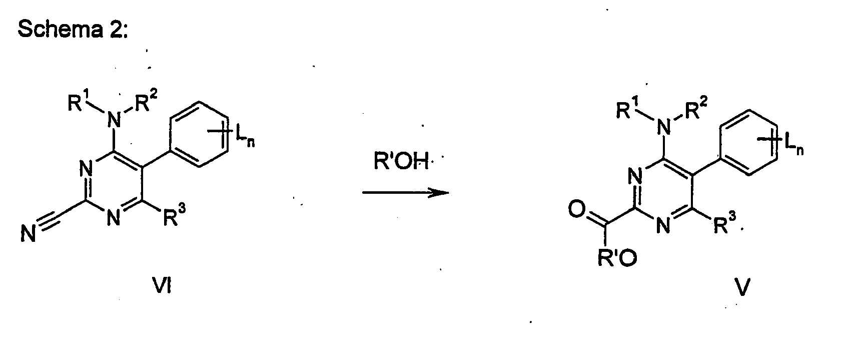

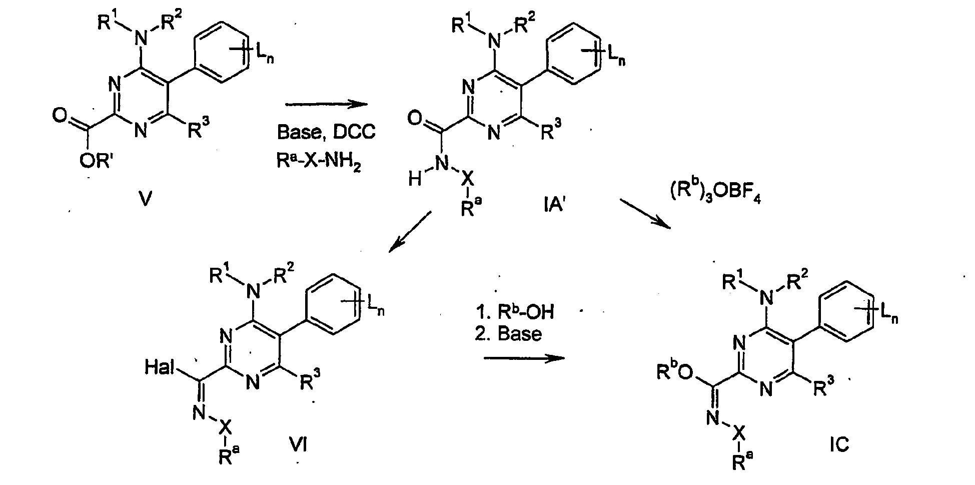

Die in Schema 2 aufgeführte Synthese der Verbindungen IA' und IC geht wiederum von Nitril IV aus. Die Nitrilbildung IV kann unter vorzugsweise sauren Bedingungen in Gegenwart von Alkoholen der Formel R'OH, wobei R' für C₁-C₈-Alkyl, C₂-C₈-Alkenyl, C₂-C₈-Alkinyl oder C₃-C₆-Cycloalkyl, wobei die Reste Alkyl, Alkenyl und Alkinyl partiell oder vollständig halogeniert sein können und eine bis drei Gruppen R^{v} tragen können, steht, hergestellt werden. Die Umsetzung von V mit Aminen zu den Amiden IA' kann wie in Org.Lett., 2001, Vol 3, S. 1053-56 oder in J.Org.Chem., 2000, Vol 85, S. 8415-20 beschrieben, durchgeführt werden. Die anschließende Umsetzung mit Meerwein Salzen der Formel (R^{b})₃OBF₄ analog den in Synth. Commun., 1983, 13, S. 753 oder Helv. Chim.Acta, 1986, 69, S. 1224 aufgeführten Vorschriften führt zu den erfindungsgemäßen Verbindungen der Formel IC. Die Iminhalogenide der Formel VI, wobei Hal für Halogen und insbesondere Chlor und Brom steht, sind analog Synthesis, 1991, Vol 9, S. 750-752 zugänglich: In einer Appel Reaktion werden beispielsweise mit Tetrabromkohlenstoff und Triphenylphosphin die entsprechenden Bromverbindungen hergestellt. Letztere lässt sich schließlich mit Alkoholen der Formel R^{b}OH und Basen zu den erfindungsgemäßen Verbindungen IC umsetzen.

Der Rest R³ (insbesondere Alkyl) in 6-Position am Pyrimidinring kann durch Umsetzung unter Übergangsmetallkatalyse, wie Ni- oder Pd-Katalyse eingeführt werden. In manchen Fällen kann es ratsam sein die Reihenfolge umzudrehen und den Substituenten R³ vor dem Substituenten NR¹R² einzuführen.

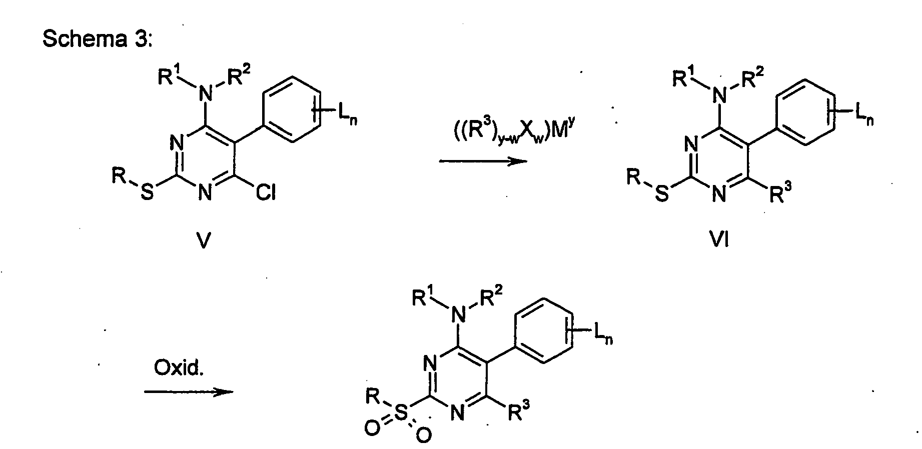

In Formel (R³)_{y-w}X_{w}-M^{y} steht M für ein Metallion der Wertigkeit Y, wie beispielsweise B, Zn, Mg, Cu oder Sn, X steht für Chlor, Brom, lod oder Hydroxy, R³ bedeutet bevorzugt C₁-C₄-Alkyl und w steht für eine Zahl von 0 bis 3. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992).

Der Substituent R^{a} in Formel IA' kann auch wie in Schema 4 gezeigt eingeführt werden.

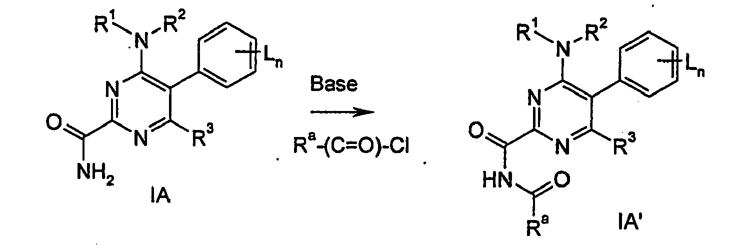

Hierbei werden die Verbindungen der Formel IA mit Hilfe starker Basen zunächst ins Anion übergeführt und anschließend mit entsprechenden Säurechloriden zu IA' umgesetzt (s. J. Chem. Soc., Perkin Trans I, 1995, S. 3043). Man gelangt so zu Verbindungen, in denen X für ein C=O -Bruckenglied steht. Als Basen für die Herstellung des Anions eignen sich beispielsweise Natriumamid und Natriumhydrid.

Die obengenannten Angaben beziehen sich insbesondere auf die Herstellung von Verbindungen, in denen R³ eine Alkylgruppe darstellt. Sofern R³ eine Cyangruppe oder einen Alkoxysubstienten bedeutet, kann der Rest R³ durch Umsetzung mit Alkalimetallcyaniden bzw. Alkalimetallalkoholaten eingeführt werden.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
**Alkyl sowie die Alkylteile von beispielsweise Alkoxy, Alkylamino, Alkoxycarbonyl:** gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl**: geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
**Alkenyl:** ungesättigte, geradkettige oder verzweigte Kohlenwasserstoffreste mit 2 bis 4, 6 oder 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1 propenyl und 1-Ethyl-2-methyl-2-propenyl;
**Alkadienyl:** ungesättigte, geradkettige oder verzweigte Kohlenwasserstoffreste mit 4, oder 6 Kohlenstoffatomen und zwei Doppelbindungen in beliebiger Position;
**Halogenalkenyl**: ungesättigte, geradkettige oder verzweigte Kohlenwasserstoffreste mit 2 bis 6 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl**: geradkettige oder verzweigte Kohlenwasserstoffgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butiriyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalkyl:** mono- oder bicyclische, gesättigte Kohlenwasserstoffgruppen mit 3 bis 6 Kohlenstoffringgliedern, z.B. C₃-C₆-Cycloalkyl wie Cyclopropyl, Cyclobutyl, Cyclopentyl, Cyclohexyl;
fünf- bis sechsgliedriger gesättigter, partiell ungesättigter oder aromatischer Heterocyclus, enthaltend ein bis vier Heteroatome aus der Gruppe O, N oder S:
- **5- oder 6-gtiedriges Heterocyclyl**, enthaltend ein bis drei Stickstoffatome und/oder ein Sauerstoff- oder Schwefelatom oder ein oder zwei Sauerstoff-und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl; 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl und 1,2,4-Hexahydrotriazin-3-yl;
- **5-gliedriges Heteroaryl,** enthaltend ein bis vier Stickstoffatome oder ein bis drei Stickstoffatome und ein Schwefel- oder Sauerstoffatom: 5-Ring Heteroarylgruppen, weiche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-lmidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
- **6-gliedriges Heteroaryl,** enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
- Ringsystem, das gegebenenfalls von R¹ und R² bzw. von A und A' zusammen mit dem Stickstoff, an den sie gebunden sind, aufgespannt wird: Pyrrolidin, Morpholin, Piperidin oder Tetrahydropyrazol.

In dem Umfang der vorliegenden Erfindung sind die (R)- und (S)-Isomere und die Racemate von Verbindungen der Formel I eingeschlossen, die chirale Zentren aufweisen.

Im folgenden werden die Ausführungsformen der Erfindung genauer beschrieben.

Im Hinblick auf die bestimmungsgemäße Verwendung der Pyrimidine der Formel I sind die folgenden Bedeutungen der Substituenten, und zwar jeweils für sich allein oder in Kombination, besonders bevorzugt:

Verbindungen I werden bevorzugt, in denen R¹ für C₁-C₆-Alkyl, C₁-C₆-Halogenalkyl, C₂-C₆-Alkenyl, C₂-C₆-Alkinyl oder C₃-C₆-Cycloalkyl und R² für Wasserstoff stehen.

Insbesondere werden Verbindungen I bevorzugt, in denen R¹ für in α-Stellung verzweigtes C₁-C₆-Alkyl, C₂-C₆-Alkenyl oder C₁-C₆-Halogenalkyl steht.

Daneben werden Verbindungen I bevorzugt, in denen R¹ für C₁-C₄-Halogenalkyl und R² für Wasserstoff stehen.

Außerdem werden Verbindungen I bevorzugt, in denen R¹ und R² zusammen mit dem Stickstoff, an das sie gebunden sind, einen fünf- oder sechsgliedrigen Ring bilden, der durch ein Sauerstoffatom unterbrochen sein kann und einen oder zwei C₁-C₆-Alkylsubstituenten tragen kann.

Insbesondere bevorzugt sind Gruppen NR¹R² wie - insbesondere in α-Stellung - methylierte Pyrrolidine oder Piperidine. Weiterhin ist 4-Methylpiperidin bevorzugt.

Insbesondere werden Pyrimidine I bevorzugt, wobei die Substituenten L¹ bis L⁵ die folgende Bedeutung haben:
- L: Halogen, Cyano, C₁-C₈-Alkyl, C₁-C₆-Alkoxy, -C(=O)-O-A, -C(=O)-N(A')A,
- A, A', A": unabhängig voneinander Wasserstoff, C₁-C₆-Alkyl, C₂-C₆-Alkenyl, C₂-C₆-Alkinyl.

Außerdem werden Pyrimidine I bevorzugt, wobei die durch Lₙ substituierte Phenylgruppe für die Gruppe B 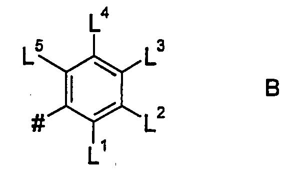 steht, worin # die Verknüpfungsstelle mit dem Pyrimidin-Gerüst ist und
- L¹: Fluor, Chlor, CH₃ oder CF₃;
- L²,L⁴: unabhängig voneinander Wasserstoff, CH₃ oder Fluor;
- L³: Wasserstoff, Fluor, Chlor, Brom, Cyano, CH₃, SCH₃, OCH₃, SO₂CH₃, CO-NH₂, CO-NHCH₃, CO-NHC₂H₅, CO-N(CH₃)₂, NH-C(=O)CH₃, N(CH₃)-C(=O)CH₃ oder COOCH₃ und
- L⁵: Wasserstoff, Fluor, Chlor oder CH₃ bedeuten.

Besonders bevorzugt werden auch Verbindungen I, in denen R³ C₁-C₄-Alkyl bedeutet, das durch Halogen substituiert sein kann.

Außerdem werden Verbindungen I besonders bevorzugt, in denen R³ für Halogen, Cyano, C₁-C₄-Alkyl oder C₁-C₄-Alkoxy steht.

Insbesondere werden Verbindungen I bevorzugt, in denen R³ Methyl, Cyano, Methoxy oder insbesondere Chlor bedeutet.

Geeignet im Hinblick auf ihre fungizide Wirkung sind Pyrimidine der Formel I, in der R⁴ für 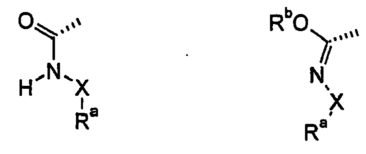 steht. Weiterhin sind Pyrimidine der Formel I bevorzugt, in der R⁴ für 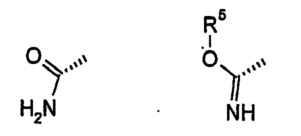 steht.

Insbesondere sind Pyrimidine der Formel bevorzugt, in der R⁴ für 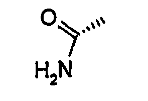 steht.

Schließlich kann R⁴ bevorzugt die folgenden Bedeutungen haben, die auch als prodrug-Restedefinitionen aufgefasst werden können (s. Medicininal Research Reviews 2003, 23, 763 - 793, oder J. of Pharmaceutical Sciences 1997, 86, 765-767):

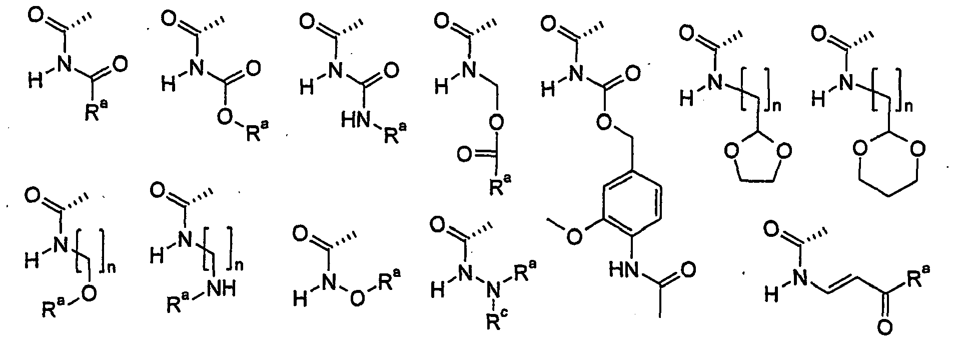

Der Index n in den Alkenylenresten der obigen Formeln steht für eine ganze Zahl 1 bis 3.

Insbesondere bevorzugt sind die Restedefinitionen R⁴:

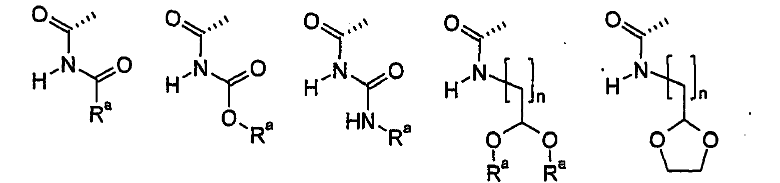

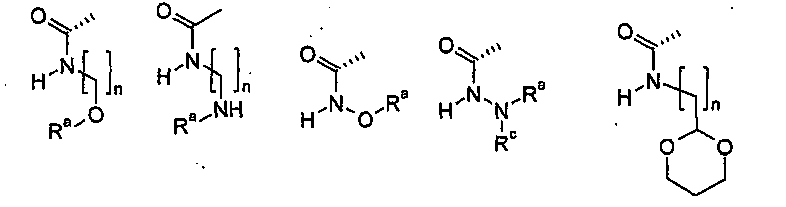

Das Brückenglied X steht bevorzugt für eine direkte Bindung und für -(C=O)-.

Der Substituent R^{a} steht vorzugsweise für Wasserstoff, Methyl, Benzyl, Trifluormethyl, Allyl, Propargyl oder Methoxymethyl und besonders bevorzugt für Wasserstoff.

Der Substituent R^{b} bedeutet bevorzugt Wasserstoff, C₁-C₆-Alkyl oder C₂-C₆-Alkenyl und insbesondere bevorzugt: Methyl, Allyl oder Propargyl.

Der Substituent R^{c} bedeutet bevorzugt Wasserstoff oder Methyl.

Die Ester der Formel V 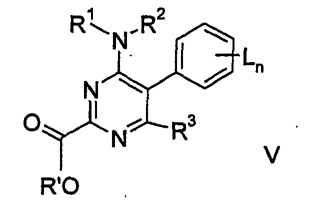 sind sowohl interessante Zwischenprodukte, als auch exzellente fungizide Wirkstoffe.

Die zuvor genannten Bevorzugungen der Definitionen von Lₙ, R¹ bis R³ gelten im gleichen Sinne auf für die Ester der Formel V.

R' steht insbesondere für einen C₁-C₆-Alkylrest, insbesondere bevorzugt für einen 1-sopropylrest.

R' kann jadoch auch die Bedeutungen Allyl, Propargyl, Benzyl, Aminoalkyl, Hydroxyalkyl, Alkoxyalkyl oder Halogenalkyl.

Insbesondere sind im Hinblick auf ihre Verwendung die in den folgenden Tabellen zusammengestellten Verbindungen I und V bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

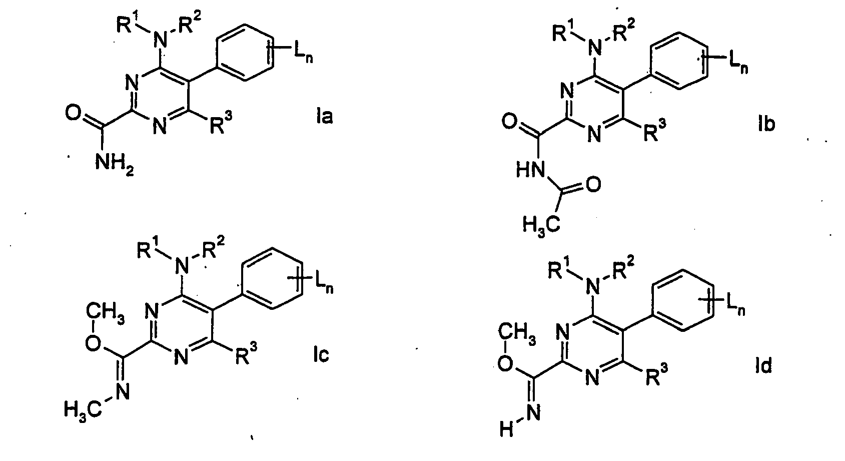

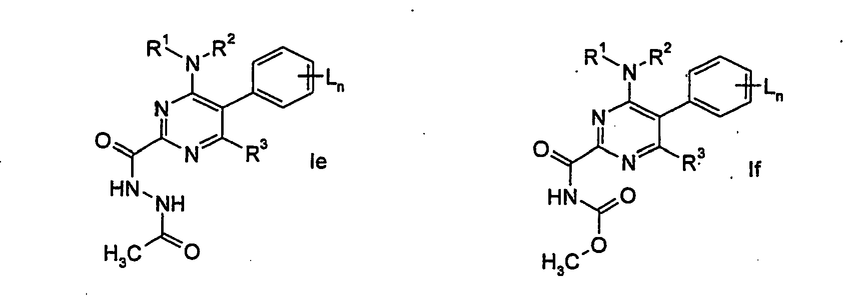

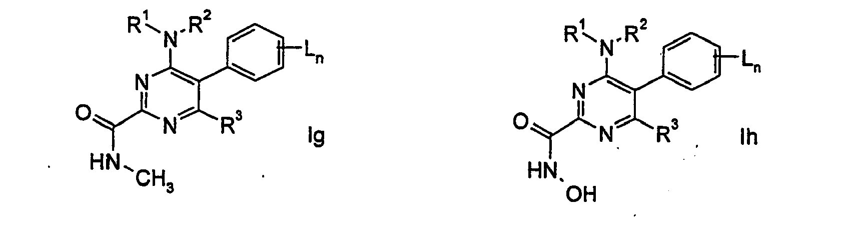

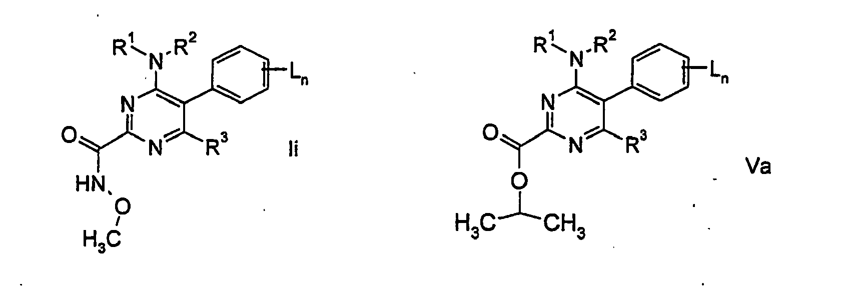

### Tabelle 1

Verbindungen der Formel Ia, Ib, Ic, Id, Ie, If, Ig, Ih, Ii und Va, in denen Lₙ 2-Fluor,6-chlor, R³ Methyl bedeuten und R¹, R² für eine Verbindung jeweils einer Zeile der Tabelle A entspricht

### Tabelle 2

Verbindungen der Formel Ia, Ib, Ic, Id, Ie, If, Ig, Ih, Ii und Va, in denen Lₙ 2,6-Difluor, R³ Methyl bedeuten und R¹, R² für eine Verbindung jeweils einer Zeile der Tabelle A entspricht

### Tabelle 3

Verbindungen der Formel Ia, Ib, Ic, Id, Ie, If, Ig, Ih, Ii und Va, in denen Lₙ 2,6-Dichlor, R³ Methyl bedeuten und R¹, R² für eine Verbindung jeweils einer Zeile der Tabelle A entspricht

### Tabelle 4

Verbindungen der Formel Ia, Ib, Ic, Id, Ie, If, Ig, Ih, Ii und Va, in denen Lₙ 2-Fluor,6-methyl, R³ Methyl bedeuten und R¹, R² für eine Verbindung jeweils einer Zeile der Tabelle A entspricht

### Tabelle 5

Verbindungen der Formel Ia, Ib, Ic, Id, Ie, If, Ig, Ih, Ii und Va, in denen Lₙ 2,4,6-Trifluor, R³ Methyl bedeuten und R¹, R² für eine Verbindung jeweils einer Zeile der Tabelle A entspricht

### Tabelle 6

Verbindungen der Formel Ia, Ib, Ic, Id, Ie, If, Ig, Ih, Ii und Va, in denen Lₙ 2-Methyl,4-fluor, R³ Methyl bedeuten und R¹, R² für eine Verbindung jeweils einer Zeile der Tabelle A entspricht

### Tabelle 7

Verbindungen der Formel Ia, Ib, Ic, Id, Ie, If, Ig, Ih, Ii und Va, in denen Lₙ 2-Fluor,4-methoxycarbonyl, R³ Methyl bedeuten und R¹, R² für eine Verbindung jeweils einer Zeile der Tabelle A entspricht

### Tabelle 8

Verbindungen der Formel Ia, Ib, Ic, Id, Ie, If, Ig, Ih, Ii und Va, in denen Lₙ 2-Fluor,4-CN, R³ Methyl bedeuten und R¹, R² für eine Verbindung jeweils einer Zeile der Tabelle A entspricht

### Tabelle 9

Verbindungen der Formel Ia, Ib, Ic, Id, Ie, If, Ig, Ih, Ii und Va, in denen Lₙ 2,4,5-Trifluor, R³ Methyl bedeuten und R¹, R² für eine Verbindung jeweils einer Zeile der Tabelle A entspricht

### Tabelle 10

Verbindungen der Formel Ia, Ib, Ic, Id, Ie, If, Ig, Ih, Ii und Va, in denen Lₙ 2,4-Dichlor, R³ Methyl bedeuten und R¹, R² für eine Verbindung jeweils einer Zeile der Tabelle A entspricht

### Tabelle 11

Verbindungen der Formel Ia, Ib, Ic, Id, Ie, If, Ig, Ih, Ii und Va, in denen Lₙ 2-Chlor, R³ Methyl bedeuten und R¹, R² für eine Verbindung jeweils einer Zeile der Tabelle A entspricht

### Tabelle 12

Verbindungen der Formel Ia, Ib, Ic, Id, Ie, If, Ig, Ih, Ii und Va, in denen Lₙ 2-Fluor, R³ Methyl bedeuten und R¹, R² für eine Verbindung jeweils einer Zeile der Tabelle A entspricht

### Tabelle 13

Verbindungen der Formel Ia, Ib, Ic, Id, Ie, If, Ig, Ih, Ii und Va, in denen Lₙ 2,4-Difluor, R³ Methyl bedeuten und R¹, R² für eine Verbindung jeweils einer Zeile der Tabelle A entspricht

### Tabelle 14

Verbindungen der Formel Ia, Ib, Ic, Id, Ie, If, Ig, Ih, Ii und Va, in denen Lₙ 2-Fluor-4-chlor, R³ Methyl bedeuten und R¹, R² für eine Verbindung jeweils einer Zeile der Tabelle A entspricht

### Tabelle 15

Verbindungen der Formel Ia, Ib, Ic, Id, Ie, If, Ig, Ih, Ii und Va, in denen Lₙ 2-Chlor-4-fluor, R³ Methyl bedeuten und R¹, R² für eine Verbindung jeweils einer Zeile der Tabelle A entspricht

### Tabelle 16

Verbindungen der Formel Ia, Ib, Ic, Id, Ie, If, Ig, Ih, Ii und Va, in denen Lₙ 2,3-Difluor, R³ Methyl bedeuten und R¹, R² für eine Verbindung jeweils einer Zeile der Tabelle A entspricht

### Tabelle 17

Verbindungen der Formel Ia, Ib, Ic, Id, Ie, If, Ig, Ih, Ii und Va, in denen Lₙ 2,5-Difluor, R³ Methyl bedeuten und R¹, R² für eine Verbindung jeweils einer Zeile der Tabelle A entspricht

### Tabelle 18

Verbindungen der Formel Ia, Ib, Ic, Id, Ie, If, Ig, Ih, Ii und Va, in denen Lₙ 2,3,4-Trifluor, R³ Methyl bedeuten und R¹, R² für eine Verbindung jeweils einer Zeile der Tabelle A entspricht

### Tabelle 19

Verbindungen der Formel Ia, Ib, Ic, Id, Ie, If, Ig, Ih, Ii und Va, in denen Lₙ 2-Methyl, R³ Methyl bedeuten und R¹, R² für eine Verbindung jeweils einer Zeile der Tabelle A entspricht

### Tabelle 20

Verbindungen der Formel Ia, Ib, Ic, Id, Ie, If, Ig, Ih, Ii und Va, in denen Lₙ 2,4-Dimethyl, R³ Methyl bedeuten und R¹, R² für eine Verbindung jeweils einer Zeile der Tabelle A entspricht

### Tabelle 21

Verbindungen der Formel Ia, Ib, Ic, Id, Ie, If, Ig, Ih, Ii und Va, in denen Lₙ 2-Methyl-4-chlor, R³ Methyl bedeuten und R¹, R² für eine Verbindung jeweils einer Zeile der Tabelle A entspricht

### Tabelle 22

Verbindungen der Formel Ia, Ib, Ic, Id, Ie, If, Ig, Ih, Ii und Va, in denen Lₙ 2-Fluor-4-methyl, R³ Methyl bedeuten und R¹, R² für eine Verbindung jeweils einer Zeile der Tabelle A entspricht

### Tabelle 23

Verbindungen der Formel Ia, Ib, Ic, Id, Ie, If, Ig, Ih, Ii und Va, in denen Lₙ 2,6-Dimethyl, R³ Methyl bedeuten und R¹, R² für eine Verbindung jeweils einer Zeile der Tabelle A entspricht

### Tabelle 24

Verbindungen der Formel Ia, Ib, Ic, Id, Ie, If, Ig, Ih, Ii und Va, in denen Lₙ 2,4,6-Trimethyl, R³ Methyl bedeuten und R¹, R² für eine Verbindung jeweils einer Zeile der Tabelle A entspricht

### Tabelle 25

Verbindungen der Formel Ia, Ib, Ic, Id, Ie, If, Ig, Ih, Ii und Va, in denen Lₙ 2,6-Difluor-4-cyano, R³ Methyl bedeuten und R¹, R² für eine Verbindung jeweils einer Zeile der Tabelle A entspricht

### Tabelle 26

Verbindungen der Formel Ia, Ib, Ic, Id, Ie, If, Ig, Ih, Ii und Va, in denen Lₙ 2,6-Difluor-4-methyl, R³ Methyl bedeuten und R¹, R² für eine Verbindung jeweils einer Zeile der Tabelle A entspricht

### Tabelle 27

Verbindungen der Formel Ia, Ib, Ic, Id, Ie, If, Ig, Ih, Ii und Va, in denen Lₙ 2,6-Difluor-4-methoxycarbonyl, R³ Methyl bedeuten und R¹, R² für eine Verbindung jeweils einer Zeile der Tabelle A entspricht

### Tabelle 28

Verbindungen der Formel Ia, Ib, Ic, Id, Ie, If, Ig, Ih, Ii und Va, in denen Lₙ 2-Chlor,4-Methoxy, R³ Methyl bedeuten und R¹, R² für eine Verbindung jeweils einer Zeile der Tabelle A entspricht

### Tabelle 29

Verbindungen der Formel Ia, Ib, Ic, Id, Ie, If, Ig, Ih, Ii und Va, in denen Lₙ 2-Chlor,4-Methyl, R³ Methyl bedeuten und R¹, R² für eine Verbindung jeweils einer Zeile der Tabelle A entspricht

### Tabelle 30

Verbindungen der Formel Ia, Ib, lc, Id, Ie, If, Ig, Ih, Ii und Va, in denen Lₙ2-Chlor,4-methoxycarbonyl, R³ Methyl bedeuten und R¹, R² für eine Verbindung jeweils einer Zeile der Tabelle A entspricht

### Tabelle 31

Verbindungen der Formel Ia, Ib, Ic, Id, Ie, If, Ig, Ih, li und Va, in denen Lₙ2-Chtor,4-Brom, R³ Methyl bedeuten und R¹, R² für eine Verbindung jeweils einer Zeile der Tabelle A entspricht

### Tabelle 32

Verbindungen der Formel Ia, Ib, Ic, Id, Ie, If, Ig, Ih, Ii und Va, in denen Lₙ 2-Chlor,4-Cyan, R³ Methyl bedeuten und R¹, R² für eine Verbindung jeweils einer Zeile der Tabelle A entspricht

### Tabelle 33

Verbindungen der Formel Ia, Ib, Ic, Id, Ie, If, Ig, Ih, Ii und Va, in denen Lₙ 2,6-Difluor,4-methoxy, R³ Methyl bedeuten und R¹, R² für eine Verbindung jeweils einer Zeile der Tabelle A entspricht

### Tabelle 34

Verbindungen der Formel Ia, Ib, Ic, Id, Ie, If, Ig, Ih, Ii und Va, in denen Lₙ2-Fluor,3-methyl, R³ Methyl bedeuten und R¹, R² für eine Verbindung jeweils einer Zeile der Tabelle A entspricht

### Tabelle 35

Verbindungen der Formel Ia, Ib, Ic, Id, Ie, If, Ig, Ih, Ii und Va, in denen Lₙ 2,5-Dimethyl, R³ Methyl bedeuten und R¹, R² für eine Verbindung jeweils einer Zeile der Tabelle A entspricht

### Tabelle 36

Verbindungen der Formel Ia, Ib, Ic, Id, Ie, If, Ig, Ih, Ii und Va, in denen Lₙ 2-Methyl,4-Cyan, R³ Methyl bedeuten und R¹, R² für eine Verbindung jeweils einer Zeile der Tabelle A entspricht

### Tabelle 37

Verbindungen der Formel Ia, Ib, Ic, Id, Ie, If, Ig, Ih, Ii und Va, in denen Lₙ 2-Methyl,4-brom, R³ Methyl bedeuten und R¹, R² für eine Verbindung jeweils einer Zeile der Tabelle A entspricht

### Tabelle 38

Verbindungen der Formel Ia, Ib, Ic, Id, Ie, If, Ig, Ih, Ii und Va, in denen Lₙ 2-Methyl,5-fluor, R³ Methyl bedeuten und R¹, R² für eine Verbindung jeweils einer Zeile der Tabelle A entspricht

### Tabelle 39

Verbindungen der Formel Ia, Ib, Ic, Id, Ie, If, Ig, Ih, Ii und Va, in denen Lₙ 2-Methyl,4-methoxy, R³ Methyl bedeuten und R¹, R² für eine Verbindung jeweils einer Zeile der Tabelle A entspricht

### Tabelle 40

Verbindungen der Formel Ia, Ib, Ic, Id, Ie, If, Ig, Ih, Ii und Va, in denen Lₙ 2-Methyl,4-methoxycarbonyl, R³ Methyl bedeuten und R¹, R² für eine Verbindung jeweils einer Zeile der Tabelle A entspricht

### Tabelle 41

Verbindungen der Formel Ia, Ib, Ic, Id, Ie, If, Ig, Ih, Ii und Va, in denen Lₙ 2,5-Dimethyl,4-brom, R³ Methyl bedeuten und R¹, R² für eine Verbindung jeweils einer Zeile der Tabelle A entspricht

### Tabelle 42

Verbindungen der Formel Ia, Ib, Ic, Id, Ie, If, Ig, Ih, Ii und Va, in denen Lₙ2-Fiuor,4-brom, R³ Methyl bedeuten und R¹, R² für eine Verbindung jeweils einer Zeile der Tabelle A entspricht

### Tabelle 43

Verbindungen der Formel Ia, Ib, Ic, Id, Ie, If, Ig, Ih, Ii und Va, in denen Lₙ 2-Fluor,4-methoxy, R² Methyl bedeuten und R¹, R² für eine Verbindung jeweils einer Zeile der Tabelle A entspricht

### Tabelle 44

Verbindungen der Formel Ia, Ib, Ic, Id, Ie, If, Ig, Ih, Ii und Va, in denen Lₙ 2-Fluor,5-methyl, R³ Methyl bedeuten und R¹, R² für eine Verbindung jeweils einer Zeile der Tabelle A entspricht

### Tabelle 45

Verbindungen der Formel Ia, Ib, Ic, Id, Ie, If, Ig, Ih, Ii und Va, in denen LₙPentafiuor, R³ Methyl bedeuten und R¹, R² für eine Verbindung jeweils einer Zeile der Tabelle A entspricht

### Tabelle 46

Verbindungen der Formel Ia, Ib, Ic, Id, Ie, If, Ig, Ih, Ii und Va, in denen Lₙ 2-Fluor,6-chlor, R³ Chlor bedeuten und R¹, R² für eine Verbindung jeweils einer Zeile der Tabelle A entspricht

### Tabelle 47

Verbindungen der Formel Ia, Ib, Ic, Id, Ie, If, Ig, Ih, Ii und Va, in denen Lₙ 2,6-Difluor, R³ Chlor bedeuten und R¹, R² für eine Verbindung jeweils einer Zeile der Tabelle A entspricht

### Tabelle 48

Verbindungen der Formel Ia, Ib, Ic, Id, Ie, If, Ig, Ih, Ii und Va, in denen Lₙ 2,6-Dichlor, R³ Chlor bedeuten und R¹, R² für eine Verbindung jeweils einer Zeile der Tabelle A entspricht

### Tabelle 49

Verbindungen der Formel Ia, Ib, Ic, Id, Ie, If, Ig, Ih, Ii und Va, in denen Lₙ 2-Fluor,6-methyl, R³ Chlor bedeuten und R¹, R² für eine Verbindung jeweils einer Zeile der Tabelle A entspricht

### Tabelle 50

Verbindungen der Formel Ia, Ib, Ic, Id, Ie, If, Ig, Ih, Ii und Va, in denen Lₙ 2,4,6-Trifluor, R³ Chlor bedeuten und R¹, R² für eine Verbindung jeweils einer Zeile der Tabelle A entspricht

### Tabelle 51

Verbindungen der Formel Ia, Ib, Ic, Id, Ie, If, Ig, Ih, Ii und Va, in denen Lₙ 2-Methyl,4-fluor, R³ Chlor bedeuten und R¹, R² für eine Verbindung jeweils einer Zeile der Tabelle A entspricht

### Tabelle 52

Verbindungen der Formel Ia, Ib, Ic, Id, Ie, If, Ig, Ih, Ii und Va, in denen Lₙ 2-Fluor,4-methoxycarbonyl, R³ Chlor bedeuten und R¹, R² für eine Verbindung jeweils einer Zeile der Tabelle A entspricht

### Tabelle 53

Verbindungen der Formel Ia, Ib, Ic, Id, Ie, If, Ig, Ih, Ii und Va, in denen Lₙ 2-Fluor,4-CN, R³ Chlor bedeuten und R¹, R² für eine Verbindung jeweils einer Zeile der Tabelle A entspricht

### Tabelle 54

Verbindungen der Formel Ia, Ib, Ic, Id, Ie, If, Ig, Ih, Ii und Va, in denen Lₙ 2,4,5-Trifluor, R³ Chlor bedeuten und R¹, R² für eine Verbindung jeweils einer Zeile der Tabelle A entspricht

### Tabelle 55

Verbindungen der Formel Ia, Ib, Ic, Id, Ie, If, Ig, Ih, Ii und Va, in denen Lₙ 2,4-Dichlor, R³ Chlor bedeuten und R¹, R² für eine Verbindung jeweils einer Zeile der Tabelle A entspricht

### Tabelle 56

Verbindungen der Formel Ia, Ib, Ic, Id, Ie, If, Ig, Ih, Ii und Va, in denen Lₙ 2-Chlor, R³ Chlor bedeuten und R¹, R² für eine Verbindung jeweils einer Zeile der Tabelle A entspricht

### Tabelle 57

Verbindungen der Formel Ia, Ib, Ic, Id, Ie, If, Ig, Ih, Ii und Va, in denen Lₙ 2-Fluor, R³ Chlor bedeuten und R¹, R² für eine Verbindung jeweils einer Zeile der Tabelle A entspricht

### Tabelle 58

Verbindungen der Formel Ia, Ib, Ic, Id, Ie, If, Ig, Ih, Ii und Va, in denen Lₙ 2,4-Difluor, R³ Chlor bedeuten und R¹, R² für eine Verbindung jeweils einer Zeile der Tabelle A entspricht

### Tabelle 59

Verbindungen der Formel Ia, Ib, Ic, Id, Ie, If, Ig, Ih, Ii und Va, in denen Lₙ 2-Fluor-4-chlor, R³ Chlor bedeuten und R¹, R² für eine Verbindung jeweils einer Zeile der Tabelle A entspricht

### Tabelle 60.

Verbindungen der Formel Ia, Ib, Ic, Id, Ie, If, Ig, Ih, Ii und Va, in denen Lₙ 2-Chlor-4-fluor, R³ Chlor bedeuten und R¹, R² für eine Verbindung jeweils einer Zeile der Tabelle A entspricht

### Tabelle 61

Verbindungen der Formel Ia, Ib, Ic, Id, Ie, If, Ig, Ih, Ii und Va, in denen Lₙ 2,3-Difluor, R³ Chlor bedeuten und R¹, R² für eine Verbindung jeweils einer Zeile der Tabelle A entspricht

### Tabelle 62

Verbindungen der Formel Ia, Ib, Ic, Id, Ie, If, Ig, Ih, Ii und Va, in denen Lₙ 2,5-Difluor, R³ Chlor bedeuten und R¹, R² für eine Verbindung jeweils einer Zeile der Tabelle A entspricht

### Tabelle 63

Verbindungen der Formel Ia, Ib, Ic, Id, Ie, If, Ig, Ih, Ii und Va, in denen Lₙ 2,3,4-Trifluor, R³ Chlor bedeuten und R¹, R² für eine Verbindung jeweils einer Zeile der Tabelle A entspricht

### Tabelle 64

Verbindungen der Formel Ia, Ib, Ic, Id, Ie, If, Ig, Ih, Ii und Va, in denen Lₙ 2-Methyl, R³ Chlor bedeuten und R¹, R² für eine Verbindung jeweils einer Zeile der Tabelle A entspricht

### Tabelle 65

Verbindungen der Formel Ia, Ib, Ic, Id, Ie, If, Ig, Ih, Ii und Va, in denen Lₙ 2,4-Dimethyl, R³ Chlor bedeuten und R¹, R² für eine Verbindung jeweils einer Zeile der Tabelle A entspricht

### Tabelle 66

Verbindungen der Formel Ia, Ib, Ic, Id, Ie, If, Ig, Ih, Ii und Va, in denen Lₙ 2-Methyl-4-chlor, R³ Chlor bedeuten und R¹, R² für eine Verbindung jeweils einer Zeile der Tabelle A entspricht

### Tabelle 67

Verbindungen der Formel Ia, Ib, Ic, Id, Ie, If, Ig, Ih, Ii und Va, in denen Lₙ 2-Fluor-4-methyl, R² Chlor bedeuten und R¹, R² für eine Verbindung jeweils einer Zeile der Tabelle A entspricht

### Tabelle 68

Verbindungen der Formel Ia, Ib, Ic, Id, Ie, If, Ig, Ih, Ii und Va, in denen Lₙ 2,6-Dimethyl, R³ Chlor bedeuten und R¹, R² für eine Verbindung jeweils einer Zeile der Tabelle A entspricht

### Tabelle 69

Verbindungen der Formel Ia, Ib, Ic, Id, Ie, If, Ig, Ih, Ii und Va, in denen Lₙ 2,4,6-Trimethyl, R³ Chlor bedeuten und R¹, R² für eine Verbindung jeweils einer Zeile der Tabelle A entspricht

### Tabelle 70

Verbindungen der Formel Ia, Ib, Ic, Id, le, If, Ig, Ih, Ii und Va, in denen Lₙ 2,6-Difluor-4-cyano, R³ Chlor bedeuten und R¹, R² für eine Verbindung jeweils einer Zeile der Tabelle A entspricht

### Tabelle 71

Verbindungen der Formel Ia, Ib, Ic, Id, Ie, If, Ig, Ih, Ii und Va, in denen Lₙ 2,6-Difluor-4-methyl, R³ Chlor bedeuten und R¹, R² für eine Verbindung jeweils einer Zeile der Tabelle A entspricht

### Tabelle 72

Verbindungen der Formel Ia, Ib, Ic, Id, Ie, If, Ig, Ih, Ii und Va, in denen Lₙ 2,6-Difluor-4-methoxycarbonyl, R³ Chlor bedeuten und R¹, R² für eine Verbindung jeweils einer Zeile der Tabelle A entspricht

### Tabelle 73

Verbindungen der Formel Ia, Ib, Ic, Id, Ie, If, Ig, Ih, Ii und Va, in denen Lₙ 2-Chlor,4-Methoxy, R³ Chlor bedeuten und R¹, R² für eine Verbindung jeweils einer Zeile der Tabelle A entspricht

### Tabelle 74

Verbindungen der Formel Ia, Ib, Ic, Id, Ie, If, Ig, Ih, Ii und Va, in denen Lₙ 2-Chlor,4-Methyl, R³ Chlor bedeuten und R¹, R² für eine Verbindung jeweils einer Zeile der Tabelle A entspricht

### Tabelle 75

Verbindungen der Formel Ia, Ib, Ic, Id, Ie, If, Ig, Ih, Ii und Va, in denen Lₙ 2-Chlor,4-methoxycarbonyl, R³ Chlor bedeuten und R¹, R² für eine Verbindung jeweils einer Zeile der Tabelle A entspricht

### Tabelle 76

Verbindungen der Formel Ia, Ib, Ic, Id, Ie, If, Ig, Ih, Ii und Va, in denen Lₙ 2-Chlor,4-Brom, R³ Chlor bedeuten und R¹, R² für eine Verbindung jeweils einer Zeile der Tabelle A entspricht

### Tabelle 77

Verbindungen der Formel Ia, Ib, Ic, Id, Ie, If, Ig, Ih, Ii und Va, in denen Lₙ 2-Chlor,4-Cyan, R³ Chlor bedeuten und R¹, R² für eine Verbindung jeweils einer Zeile der Tabelle A entspricht

### Tabelle 78

Verbindungen der Formel Ia, Ib, Ic, Id, Ie, If, Ig, Ih, Ii und Va, in denen Lₙ 2,6-Difluor,4-methoxy, R³ Chlor bedeuten und R¹, R² für eine Verbindung jeweils einer Zeile der Tabelle A entspricht

### Tabelle 79

Verbindungen der Formel Ia, Ib, Ic, Id, le, If, Ig, Ih, Ii und Va, in denen Lₙ2-Fiuor,3-methyl, R³ Chlor bedeuten und R¹, R² für eine Verbindung jeweils einer Zeile der Tabelle A entspricht

### Tabelle 80

Verbindungen der Formel Ia, Ib, Ic, Id, Ie, If, Ig, Ih, Ii und Va, in denen Lₙ 2,5-Dimethyl, R³ Chlor bedeuten und R¹, R² für eine Verbindung jeweils einer Zeile der Tabelle A entspricht

### Tabelle 81

Verbindungen der Formel Ia, Ib, Ic, Id, Ie, If, Ig, Ih, Ii und Va, in denen Lₙ 2-Methyl,4-cyan, R³ Chlor bedeuten und R¹, R² für eine Verbindung jeweils einer Zeile der Tabelle A entspricht

### Tabelle 82

Verbindungen der Formel Ia, Ib, Ic, Id, Ie, If, Ig, Ih, Ii und Va, in denen Lₙ 2-Methyl,4-brom, R³ Chlor bedeuten und R¹, R² für eine Verbindung jeweils einer Zeile der Tabelle A entspricht

### Tabelle 83

Verbindungen der Formel Ia, Ib, Ic, Id, Ie, If, Ig, Ih, Ii und Va, in denen Lₙ2-Methyl,5-fluor, R³ Chlor bedeuten und R¹, R² für eine Verbindung jeweils einer Zeile der Tabelle A entspricht

### Tabelle 84

Verbindungen der Formel Ia, Ib, Ic, Id, Ie, If, Ig, Ih, Ii und Va, in denen Lₙ2-Methyl,4-methoxy, R³ Chlor bedeuten und R¹, R² für eine Verbindung jeweils einer Zeile der Tabelle A entspricht

### Tabelle 85

Verbindungen der Formel Ia, Ib, Ic, Id, Ie, If, Ig, Ih, Ii und Va, in denen Lₙ 2-Methyl,4-methoxycarbonyl, R³ Chlor bedeuten und R¹, R² für eine Verbindung jeweils einer Zeile der Tabelle A entspricht

### Tabelle 86

Verbindungen der Formel Ia, Ib, Ic, Id, Ie, If, Ig, Ih, Ii und Va, in denen Lₙ2,5-Dimethyl,4-brom, R³ Chlor bedeuten und R¹, R² für eine Verbindung jeweils einer Zeile der Tabelle A entspricht

### Tabelle 87

Verbindungen der Formel Ia, Ib, Ic, Id, Ie, If, Ig, Ih, Ii und Va, in denen Lₙ2-Fluor,4-brom, R³ Chlor bedeuten und R¹, R² für eine Verbindung jeweils einer Zeile der Tabelle A entspricht

### Tabelle 88

Verbindungen der Formel Ia, Ib, Ic, Id, Ie, If, Ig, Ih, Ii und Va, in denen Lₙ 2-Fluor,4-methoxy, R³ Chlor bedeuten und R¹, R² für eine Verbindung jeweils einer Zeile der Tabelle A entspricht

### Tabelle 89

Verbindungen der Formel Ia, Ib, Ic, Id, Ie, If, Ig, Ih, Ii und Va, in denen Lₙ 2-Fluor,5-methyl, R³ Chlor bedeuten und R¹, R² für eine Verbindung jeweils einer Zeile der Tabelle A entspricht

### Tabelle 90

Verbindungen der Formel Ia, Ib, Ic, Id, Ie, If, Ig, Ih, Ii und Va, in denen Lₙ Pentafluor, R³ Chlor bedeuten und R¹, R² für eine Verbindung jeweils einer Zeile der Tabelle A entspricht

### Tabelle 91

Verbindungen der Formel Ia, Ib, Ic, Id, Ie, If, Ig, Ih, Ii und Va, in denen Lₙ 2-Fluor,6-chlor, R³ Methoxy bedeuten und R¹, R² für eine Verbindung jeweils einer Zeile der Tabelle A entspricht

### Tabelle 92

Verbindungen der Formel Ia, Ib, Ic, Id, Ie, If, Ig, Ih, Ii und Va, in denen Lₙ 2,6-Difluor, R³ Methoxy bedeuten und R¹, R² für eine Verbindung jeweils einer Zeile der Tabelle A entspricht

### Tabelle 93

Verbindungen der Formel Ia, Ib, Ic, Id, Ie, If, Ig, Ih, Ii und Va, in denen Lₙ 2,6-Dichlor, R³ Methoxy bedeuten und R¹, R² für eine Verbindung jeweils einer Zeile der Tabelle A entspricht

### Tabelle 94

Verbindungen der Formel Ia, Ib, Ic, Id, Ie, If, Ig, Ih, Ii und Va, in denen Lₙ 2-Fluor,6-methyl, R³ Methoxy bedeuten und R¹, R² für eine Verbindung jeweils einer Zeile der Tabelle A entspricht

### Tabelle 95

Verbindungen der Formel Ia, Ib, Ic, Id, Ie, If, Ig, Ih, Ii und Va, in denen Lₙ 2,4,6-Trifluor, R³ Methoxy bedeuten und R¹, R² für eine Verbindung jeweils einer Zeile der Tabelle A entspricht

### Tabelle 96

Verbindungen der Formel Ia, Ib, Ic, Id, Ie, If, Ig, Ih, Ii und Va, in denen Lₙ 2-Methyl,4-fluor, R³ Methoxy bedeuten und R¹, R² für eine Verbindung jeweils einer Zeile der Tabelle A entspricht

### Tabelle 97

Verbindungen der Formel Ia, Ib, Ic, Id, Ie, If, Ig, Ih, Ii und Va, in denen Lₙ 2-Fluor,4-methoxycarbonyl, R³ Methoxy bedeuten und R¹, R² für eine Verbindung jeweils einer Zeile der Tabelle A entspricht

### Tabelle 98

Verbindungen der Formel Ia, Ib, Ic, Id, Ie, If, Ig, Ih, Ii und Va, in denen Lₙ 2-Fluor,4-CN, R³ Methoxy bedeuten und R¹, R² für eine Verbindung jeweils einer Zeile der Tabelle A entspricht

### Tabelle 99

Verbindungen der Formel Ia, Ib, Ic, Id, Ie, If, Ig, Ih, Ii und Va, in denen Lₙ 2,4,5-Trifluor, R³ Methoxy bedeuten und R¹, R² für eine Verbindung jeweils einer Zeile der Tabelle A entspricht

### Tabelle 100

Verbindungen der Formel Ia, Ib, Ic, Id, Ie, If, Ig, Ih, Ii und Va, in denen Lₙ 2,4-Dichlor, R³ Methoxy bedeuten und R¹, R² für eine Verbindung jeweils einer Zeile der Tabelle A entspricht

### Tabelle 101

Verbindungen der Formel Ia, Ib, Ic, Id, Ie, If, Ig, Ih, Ii und Va, in denen Lₙ 2-Chlor, R³ Methoxy bedeuten und R¹, R² für eine Verbindung jeweils einer Zeile der Tabelle A entspricht

### Tabelle 102

Verbindungen der Formel Ia, Ib, Ic, Id, Ie, If, Ig, Ih, Ii und Va, in denen Lₙ 2-Fluor, R³ Methoxy bedeuten und R¹, R² für eine Verbindung jeweils einer Zeile der Tabelle A entspricht

### Tabelle 103

Verbindungen der Formel Ia, Ib, Ic, Id, Ie, If, Ig, Ih, Ii und Va, in denen Lₙ 2,4-Difluor, R³ Methoxy bedeuten und R¹, R² für eine Verbindung jeweils einer Zeile der Tabelle A entspricht

### Tabelle 104

Verbindungen der Formel Ia, Ib, Ic, Id, Ie, If, Ig, Ih, Ii und Va, in denen Lₙ 2-Fluor-4-chlor, R³ Methoxy bedeuten und R¹, R² für eine Verbindung jeweils einer Zeile der Tabelle A entspricht

### Tabelle 105

Verbindungen der Formel Ia, Ib, Ic, Id, Ie, If, Ig, Ih, Ii und Va, in denen Lₙ 2-Chlor-4-fluor, R³ Methoxy bedeuten und R¹, R² für eine Verbindung jeweils einer Zeile der Tabelle A entspricht

### Tabelle 106

Verbindungen der Formel Ia, Ib, Ic, Id, Ie, If, Ig, Ih, Ii und Va, in denen Lₙ 2,3-Difluor, R³ Methoxy bedeuten und R¹, R² für eine Verbindung jeweils einer Zeile der Tabelle A entspricht

### Tabelle 107

Verbindungen der Formel Ia, Ib, Ic, Id, Ie, If, Ig, Ih, Ii und Va, in denen Lₙ 2,5-Difluor, _{R}³ Methoxy bedeuten und R¹, R² für eine Verbindung jeweils einer Zeile der Tabelle A entspricht

### Tabelle 108

Verbindungen der Formel Ia, Ib, Ic, Id, Ie, If, Ig, Ih, Ii und Va, in denen Lₙ 2,3,4-Trifluor, R³ Methoxy bedeuten und R¹, R² für eine Verbindung jeweils einer Zeile der Tabelle A entspricht

### Tabelle 109

Verbindungen der Formel Ia, Ib, Ic, Id, Ie, If, Ig, Ih, Ii und Va, in denen Lₙ 2-Methyl, R³ Methoxy bedeuten und R¹, R² für eine Verbindung jeweils einer Zeile der Tabelle A entspricht

### Tabelle 110

Verbindungen der Formel Ia, Ib, Ic, Id, Ie, If, Ig, Ih, Ii und Va, in denen Lₙ 2,4-Dimethyl, R³ Methoxy bedeuten und R¹, R² für eine Verbindung jeweils einer Zeile der Tabelle A entspricht

### Tabelle 111

Verbindungen der Formel Ia, Ib, Ic, Id, Ie, If, Ig, Ih, Ii und Va, in denen Lₙ 2-Methyl-4-chlor, R³ Methoxy bedeuten und R¹, R² für eine Verbindung jeweils einer Zeile der Tabelle A entspricht

### Tabelle 112

Verbindungen der Formel Ia, Ib, Ic, Id, Ie, If, Ig, Ih, Ii und Va, in denen Lₙ 2-Fluor-4-methyl, R³ Methoxy bedeuten und R¹, R² für eine Verbindung jeweils einer Zeile der Tabelle A entspricht

### Tabelle 113

Verbindungen der Formel Ia, Ib, Ic, Id, Ie, If, Ig, Ih, Ii und Va, in denen Lₙ 2,6-Dimethyl, R³ Methoxy bedeuten und R¹, R² für eine Verbindung jeweils einer Zeile der Tabelle A entspricht

### Tabelle 114

Verbindungen der Formel Ia, Ib, Ic, Id, Ie, If, Ig, Ih, Ii und Va, in denen Lₙ 2,4,6-Trimethyl, R³ Methoxy bedeuten und R¹, R² für eine Verbindung jeweils einer Zeile der Tabelle A entspricht

### Tabelle 115.

Verbindungen der Formel la, Ib, Ic, Id, Ie, If, Ig, Ih, Ii und Va, in denen Lₙ 2,6-Difluor-4-cyano, R³ Methoxy bedeuten und R¹, R² für eine Verbindung jeweils einer Zeile der Tabelle A entspricht

### Tabelle 116

Verbindungen der Formel Ia, Ib, Ic, Id, Ie, If, Ig, Ih, Ii und Va, in denen Lₙ 2,6-Difluor-4-methyl, R³ Methoxy bedeuten und R¹,R² für eine Verbindung jeweils einer Zeile der Tabelle A entspricht

### Tabelle 117

Verbindungen der Formel Ia, Ib, Ic, Id, Ie, If, Ig, Ih, Ii und Va, in denen Lₙ 2,6-Difluor-4-Methoxycarbonyl, R³ Methoxy bedeuten und R¹, R² für eine Verbindung jeweils einer Zeile der Tabelle A entspricht

### Tabelle 118

Verbindungen der Formel Ia, Ib, Ic, Id, Ie, If, Ig, Ih, Ii und Va, in denen Lₙ 2-Chlor,4-Methoxy, R³ Methoxy bedeuten und R¹, R² für eine Verbindung jeweils einer Zeile der Tabelle A entspricht

### Tabelle 119

Verbindungen der Formel Ia, Ib, Ic, Id, Ie, If, Ig, Ih, Ii und Va, in denen Lₙ 2-Chlor,4-Methyl, R³ Methoxy bedeuten und R¹, R² für eine Verbindung jeweils einer Zeile der Tabelle A entspricht

### Tabelle 120

Verbindungen der Formel Ia, Ib, Ic, Id, Ie, If, Ig, Ih, Ii und Va, in denen Lₙ 2-Chlor,4-methoxycarbonyl, R³ Methoxy bedeuten und R¹, R² für eine Verbindung jeweils einer Zelle der Tabelle A entspricht

### Tabelle 121

Verbindungen der Formel Ia, Ib, Ic, Id, Ie, If, Ig, Ih, Ii und Va, in denen Lₙ 2-Chlor,4-Methoxy, R³ Methoxy bedeuten und R¹, R² für eine Verbindung jeweils einer Zeile der Tabelle A entspricht

### Tabelle 122

Verbindungen der Formel Ia, Ib, Ic, Id, Ie, If, Ig, Ih, Ii und Va, in denen Lₙ 2-Chlor,4-Cyan, R³ Methoxy bedeuten und R¹, R² für eine Verbindung jeweils einer Zeile der Tabelle A entspricht

### Tabelle 123

Verbindungen der Formel Ia, Ib, Ic, Id, Ie, If, Ig, Ih, Ii und Va, in denen Lₙ 2,6-Difluor, 4-methoxy, R³ Methoxy bedeuten und R¹, R² für eine Verbindung jeweils einer Zeile der Tabelle A entspricht

### Tabelle 124

Verbindungen der Formel Ia, Ib, Ic, Id, Ie, If, Ig, Ih, Ii und Va, in denen Lₙ 2-Fluor,3-methyl, R³ Methoxy bedeuten und R¹, R² für eine Verbindung jeweils einer Zeile der Tabelle A entspricht

### Tabelle 125

Verbindungen der Formel Ia, Ib, Ic, Id, Ie, If, Ig, Ih, Ii und Va, in denen Lₙ 2,5-Dimethyl, R³ Methoxy bedeuten und R¹, R² für eine Verbindung jeweils einer Zeile der Tabelle A entspricht

### Tabelle 126

Verbindungen der Formel Ia, Ib, Ic, Id, Ie, If, Ig, Ih, Ii und Va, in denen Lₙ 2-Methyl,4-Cyan, R³ Methoxy bedeuten und R¹, R² für eine Verbindung jeweils einer Zeile der Tabelle A entspricht

### Tabelle 127

Verbindungen der Formel Ia, Ib, Ic, Id, Ie, If, Ig, Ih, Ii und Va, in denen Lₙ2-Methyl,4-Brom, R³ Methoxy bedeuten und R¹, R² für eine Verbindung jeweils einer Zeile der Tabelle A entspricht

### Tabelle 128

Verbindungen der Formel Ia, Ib, Ic, Id, Ie, If, Ig, Ih, Ii und Va, in denen Lₙ 2-Methyl,5-fluor, R³ Methoxy bedeuten und R¹, R² für eine Verbindung jeweils einer Zeile der Tabelle A entspricht

### Tabelle 129

Verbindungen der Formel Ia, Ib, Ic, Id, Ie, If, Ig, Ih, Ii und Va, in denen Lₙ 2-Methyl,4-methoxy, R³ Methoxy bedeuten und R¹, R² für eine Verbindung jeweils einer Zeile der Tabelle A entspricht

### Tabelle 130

Verbindungen der Formel Ia, Ib, Ic, Id, Ie, If, Ig, Ih, Ii und Va, in denen Lₙ 2-Methyl,4-methoxycarbonyl, R³ Methoxy bedeuten und R¹, R² für eine Verbindung jeweils einer Zeile der Tabelle A entspricht

### Tabelle 131

Verbindungen der Formel Ia, Ib, Ic, Id, Ie, If, Ig, Ih, Ii und Va, in denen Lₙ 2,5-Dimethyl,4-brom, R³ Methoxy bedeuten und R¹, R² für eine Verbindung jeweils einer Zeile der Tabelle A entspricht

### Tabelle 132

Verbindungen der Formel Ia, Ib, Ic, Id, Ie, If, Ig, Ih, Ii und Va, in denen Lₙ 2-Fluor,4-brom, R³ Methoxy bedeuten und R¹, R² für eine Verbindung jeweils einer Zeile der Tabelle A entspricht

### Tabelle 133

Verbindungen der Formel Ia, Ib, Ic, Id, Ie, If, Ig, Ih, Ii und Va, in denen Lₙ 2-Fluor,4-methoxy, R³ Methoxy bedeuten und R¹, R² für eine Verbindung jeweils einer Zeile der Tabelle A entspricht

### Tabelle 134

Verbindungen der Formel Ia, Ib, Ic, Id, Ie, If, Ig, Ih, Ii und Va, in denen Lₙ2-Fluor,5-methyl, R³ Methoxy bedeuten und R¹, R² für eine Verbindung jeweils einer Zeile der Tabelle A entspricht

### Tabelle 135

Verbindungen der Formel Ia, Ib, Ic, Id, Ie, If, Ig, Ih, Ii und Va, in denen Lₙ Pentafluor, R³ Methoxy bedeuten und R¹, R² für eine Verbindung jeweils einer Zeile der Tabelle A entspricht

### Tabelle 136

Verbindungen der Formel Ia, Ib, Ic, Id, Ie, If, Ig, Ih, Ii und Va, in denen Lₙ 2-Fluor,6-chlor, R³ Cyano bedeuten und R¹, R² für eine Verbindung jeweils einer Zeile der Tabelle A entspricht

### Tabelle 137

Verbindungen der Formel Ia, Ib, Ic, Id, Ie, If, Ig, Ih, Ii und Va, in denen Lₙ 2,6-Difluor, R³ Cyano bedeuten und R¹, R² für eine Verbindung jeweils einer Zeile der Tabelle A entspricht

### Tabelle 138

Verbindungen der Formel Ia, Ib, Ic, Id, Ie, If, Ig, Ih, Ii und Va, in denen Lₙ 2,6-Dichlor, R³ Cyano bedeuten und R¹, R² für eine Verbindung jeweils einer Zeile der Tabelle A entspricht

### Tabelle 139

Verbindungen der Formel Ia, Ib, Ic, Id, Ie, If, Ig, Ih, Ii und Va, in denen Lₙ 2-Fluor,6-methyl, R³ Cyano bedeuten und R¹, R² für eine Verbindung jeweils einer Zeile der Tabelle A entspricht

### Tabelle 140

Verbindungen der Formel Ia, Ib, Ic, Id, Ie, If, Ig, Ih, Ii und Va, in denen Lₙ 2,4,6-Trifluor, R³ Cyano bedeuten und R¹, R² für eine Verbindung jeweils einer Zeile der Tabelle A entspricht

### Tabelle 141

Verbindungen der Formel Ia, Ib, Ic, Id, Ie, If, Ig, Ih, Ii und Va, in denen Lₙ 2-Methyl,4-fluor, R³ Cyano bedeuten und R¹, R² für eine Verbindung jeweils einer Zeile der Tabelle A entspricht

### Tabelle 142

Verbindungen der Formel Ia, Ib, Ic, Id, Ie, If, Ig, Ih, Ii und Va, in denen Lₙ 2-Fluor,4-methoxycarbonyl, R³ Cyano bedeuten und R¹, R² für eine Verbindung jeweils einer Zeile der Tabelle A entspricht

### Tabelle 143

Verbindungen der Formel Ia, Ib, Ic, Id, Ie, If, Ig, Ih, Ii und Va, in denen Lₙ 2-Fluor,4-CN, R³ Cyano bedeuten und R¹, R² für eine Verbindung jeweils einer Zeile der Tabelle A entspricht

### Tabelle 144

Verbindungen der Formel Ia, Ib, Ic, Id, Ie, If, Ig, Ih, Ii und Va, in denen Lₙ 2,4,5-Trifluor, R³ Cyano bedeuten und R¹, R² für eine Verbindung jeweils einer Zeile der Tabelle A entspricht

### Tabelle 145

Verbindungen der Formel Ia, Ib, Ic, Id, Ie, If, Ig, Ih, Ii und Va, in denen Lₙ 2,4-Dichlor, R³ Cyano bedeuten und R¹, R² für eine Verbindung jeweils einer Zeile der Tabelle A entspricht

### Tabelle 146

Verbindungen der Formel Ia, Ib, Ic, Id, Ie, If, Ig, Ih, Ii und Va, in denen Lₙ 2-Chlor, R³ Cyano bedeuten und R¹, R² für eine Verbindung jeweils einer Zeile der Tabelle A entspricht

### Tabelle 147

Verbindungen der Formel Ia, Ib, Ic, Id, Ie, If, Ig, Ih, Ii und Va, in denen Lₙ 2-Fluor, R³ Cyano bedeuten und R¹, R² für eine Verbindung jeweils einer Zeile der Tabelle A entspricht

### Tabelle 148

Verbindungen der Formel Ia, Ib, Ic, Id, Ie, If, Ig, Ih, Ii und Va, in denen Lₙ 2,4-Difluor, R³ Cyano bedeuten und R¹, R² für eine Verbindung jeweils einer Zeile der Tabelle A entspricht

### Tabelle 149

Verbindungen der Formel Ia, Ib, Ic, Id, Ie, If, Ig, Ih, Ii und Va, in denen Lₙ 2-Fluor-4-chlor, R³ Cyano bedeuten und R¹, R² für eine Verbindung jeweils einer Zeile der Tabelle A entspricht

### Tabelle 150

Verbindungen der Formel Ia, Ib, Ic, Id, Ie, If, Ig, Ih, Ii und Va, in denen Lₙ 2-Chlor-4-fluor, R³ Cyano bedeuten und R¹, R² für eine Verbindung jeweils einer Zeile der Tabelle A entspricht

### Tabelle 151

Verbindungen der Formel Ia, Ib, Ic, Id, Ie, If, Ig, Ih, Ii und Va, in denen Lₙ 2,3-Difluor, R³ Cyano bedeuten und R¹, R² für eine Verbindung jeweils einer Zeile der Tabelle A entspricht

### Tabelle 152

Verbindungen der Formel Ia, Ib, Ic, Id, Ie, If, Ig, Ih, Ii und Va, in denen Lₙ 2,5-Difluor, R³ Cyano bedeuten und R¹, R² für eine Verbindung jeweils einer Zeile der Tabelle A entspricht

### Tabelle 153

Verbindungen der Formel Ia, Ib, Ic, Id, Ie, If, Ig, Ih, Ii und Va, in denen Lₙ 2,3,4-Trifluor, R³ Cyano bedeuten und R¹, R² für eine Verbindung jeweils einer Zeile der Tabelle A entspricht

### Tabelle 154

Verbindungen der Formel Ia, Ib, Ic, Id, Ie, If, Ig, Ih, Ii und Va, in denen Lₙ 2-Methyl, R³ Cyano bedeuten und R¹, R² für eine Verbindung jeweils einer Zeile der Tabelle A entspricht

### Tabelle 155

Verbindungen der Formel Ia, Ib, Ic, Id, Ie, If, Ig, Ih, Ii und Va, in denen Lₙ 2,4-Dimethyl, R³ Cyano bedeuten und R¹, R² für eine Verbindung jeweils einer Zeile der Tabelle A entspricht

### Tabelle 156

Verbindungen der Formel Ia, Ib, Ic, Id, Ie, If, Ig, Ih, Ii und Va, in denen Lₙ 2-Methyl-4-chlor, R³ Cyano bedeuten und R¹, R² für eine Verbindung jeweils einer Zeile der Tabelle A entspricht

### Tabelle 157

Verbindungen der Formel Ia, Ib, Ic, Id, Ie, If, Ig, Ih, Ii und Va, in denen Lₙ 2-Fluor-4-methyl, R³ Cyano bedeuten und R¹, R² für eine Verbindung jeweils einer Zeile der Tabelle A entspricht

### Tabelle 158

Verbindungen der Formel Ia, Ib, Ic, Id, le, If, Ig, Ih, Ii und Va, in denen Lₙ 2,6-Dimethyl, R³ Cyano bedeuten und R¹, R² für eine Verbindung jeweils einer Zeile der Tabelle A entspricht

### Tabelle 159

Verbindungen der Formel Ia, Ib, Ic, Id, Ie, If, Ig, Ih, Ii und Va, in denen Lₙ 2,4,6-Trimethyl, R³ Cyano bedeuten und R¹, R² für eine Verbindung jeweils einer Zeile der Tabelle A entspricht

### Tabelle 160

Verbindungen der Formel Ia, Ib, Ic, Id, Ie, If, Ig, Ih, Ii und Va, in denen Lₙ 2,6-Difluor-4-cyano, R³ Cyano bedeuten und R¹, R² für eine Verbindung jeweils einer Zeile der Tabelle A entspricht

### Tabelle 161

Verbindungen der Formel Ia, Ib, Ic, Id, Ie, If, Ig, Ih, Ii und Va, in denen Lₙ 2,6-Difluor-4-methyl, R³ Cyano bedeuten und R¹, R² für eine Verbindung jeweils einer Zeile der Tabelle A entspricht

### Tabelle 162

Verbindungen der Formel Ia, Ib, Ic, Id, Ie, If, Ig, Ih, Ii und Va, in denen Lₙ 2,6-Difluor-4-methoxycarbonyl, R³ Cyano bedeuten und R¹, R² für eine Verbindung jeweils einer Zeile der Tabelle A entspricht

### Tabelle 163

Verbindungen der Formel Ia, Ib, Ic, Id, Ie, If, Ig, Ih, Ii und Va, in denen Lₙ 2-Chlor,4-Methoxy, R³ Cyano bedeuten und R¹, R² für eine Verbindung jeweils einer Zeile der Tabelle A entspricht

### Tabelle 164

Verbindungen der Formel Ia, Ib, Ic, Id, Ie, If, Ig, Ih, Ii und Va, in denen Lₙ 2-Chlor,4-Methyl, R³ Cyano bedeuten und R¹, R² für eine Verbindung jeweils einer Zeile der Tabelle A entspricht

### Tabelle 165

Verbindungen der Formel Ia, Ib, Ic, Id, Ie, If, Ig, Ih, Ii und Va, in denen Lₙ 2-Chlor,4-methoxycarbonyl, R³ Cyano bedeuten und R¹, R² für eine Verbindung jeweils einer Zeile der Tabelle A entspricht

### Tabelle 166

Verbindungen der Formel Ia, Ib, Ic, Id, Ie, If, Ig, Ih, Ii und Va, in denen Lₙ 2-Chlor,4-Brom, R³ Cyano bedeuten und R¹, R² für eine Verbindung jeweils einer Zeile der Tabelle A entspricht

### Tabelle 167

Verbindungen der Formel Ia, Ib, Ic, Id, Ie, If, Ig, Ih, Ii und Va, in denen Lₙ 2-Chlor,4-Cyan, R³ Cyano bedeuten und R¹, R² für eine Verbindung jeweils einer Zeile der Tabelle A entspricht

### Tabelle 168

Verbindungen der Formel Ia, Ib, Ic, Id, Ie, If, Ig, Ih, Ii und Va, in denen Lₙ 2,6-Difluor,4-methoxy, R³ Cyano bedeuten und R¹, R² für eine Verbindung jeweils einer Zeile der Tabelle A entspricht

### Tabelle 169

Verbindungen der Formel Ia, Ib, Ic, Id, Ie, If, Ig, Ih, Ii und Va, in denen Lₙ2-Fluor,3-methyl, R³ Cyano bedeuten und R¹, R² für eine Verbindung jeweils einer Zeile der Tabelle A entspricht

### Tabelle 170

Verbindungen der Formel Ia, Ib, Ic, Id, Ie, If, Ig, Ih, Ii und Va, in denen Lₙ 2,5-Dimethyl, R³ Cyano bedeuten und R¹, R² für eine Verbindung jeweils einer Zeile der Tabelle A entspricht

### Tabelle 171

Verbindungen der Formel Ia, Ib, Ic, Id, Ie, If, Ig, Ih, Ii und Va, in denen Lₙ 2-Methyl,4-cyan, R³ Cyano bedeuten und R¹, R² für eine Verbindung jeweils einer Zeile der Tabelle A entspricht

### Tabelle 172

Verbindungen der Formel Ia, Ib, Ic, Id, Ie, If, Ig, Ih, Ii und Va, in denen Lₙ2-Methyl,4-brom, R³ Cyano bedeuten und R¹, R² für eine Verbindung jeweils einer Zeile der Tabelle A entspricht

### Tabelle 173

Verbindungen der Formel Ia, Ib, Ic, Id, Ie, If, Ig, Ih, Ii und Va, in denen Lₙ2-Methyl,5-fluor, R³ Cyano bedeuten und R¹, R² für eine Verbindung jeweils einer Zeile der Tabelle A entspricht

### Tabelle 174

Verbindungen der Formel Ia, Ib, Ic, Id, Ie, If, Ig, Ih, Ii und Va, in denen Lₙ 2-Methyl,4-methoxy, R³ Cyano bedeuten und R¹, R² für eine Verbindung jeweils einer Zeile der Tabelle A entspricht

### Tabelle 175

Verbindungen der Formel Ia, Ib, Ic, Id, Ie, If, Ig, Ih, Ii und Va, in denen Lₙ 2-Methyl,4-methoxycarbonyl, R³ Cyano bedeuten und R¹, R² für eine Verbindung jeweils einer Zeile der Tabelle A entspricht

### Tabelle 176

Verbindungen der Formel Ia, Ib, Ic, Id, Ie, If, Ig, Ih, Ii und Va, in denen Lₙ2,5-Dimethyl,4-brom, R³ Cyano bedeuten und R¹, R² für eine Verbindung jeweils einer Zeile der Tabelle A entspricht

### Tabelle 177

Verbindungen der Formel Ia, Ib, Ic, Id, Ie, If, Ig, Ih, Ii und Va, in denen Lₙ 2-Fluor,4-brom, R³ Cyano bedeuten und R¹, R² für eine Verbindung jeweils einer Zeile der Tabelle A entspricht

### Tabelle 178

Verbindungen der Formel Ia, Ib, Ic, Id, Ie, If, Ig, Ih, Ii und Va, in denen Lₙ 2-Fluor,4-methoxy, R³ Cyano bedeuten und R¹, R² für eine Verbindung jeweils einer Zeile der Tabelle. A entspricht

### Tabelle 179

Verbindungen der Formel Ia, Ib, Ic, Id, Ie, If, Ig, Ih, Ii und Va, in denen Lₙ2-Fluor,5-methyl, R³ Cyano bedeuten und R¹, R² für eine Verbindung jeweils einer Zeile der Tabelle A entspricht

### Tabelle 180

Verbindungen der Formel Ia, Ib, Ic, Id, Ie, If, Ig, Ih, Ii und Va, in denen Lₙ Pentafluor, R³ Cyano bedeuten und R¹, R² für eine Verbindung jeweils einer Zeile der Tabelle A entspricht

**Tabelle A**

| **No.** | **R¹** | **R²** |
|---|---|---|
| A-1 | CH₂CH₃ | H |
| A-2 | CH₂CH₃ | CH₃ |
| A-3 | CH₂CH₃ | CH₂CH₃ |
| A-4 | CH₂CH₂CH₃ | H |
| A-5 | CH₂CH₂CH₃ | CH₃ |
| A-6 | CH₂CH₂CH₃ | CH₂CH₃ |
| A-7 | CH₂CH₂CH₃ | CH₂CH₂CH₃ |
| A-8 | CH₂CH₂F | H |
| A-9 | CH₂CH₂F | CH₃ |
| A-10 | CH₂CH₂F | CH₂CH₃ |
| A-11 | CH₂CF₃ | H |
| A-12 | CH₂CF₃ | CH₃ |
| A-13 | CH₂CF₃ | CH₂CH₃ |
| A-14 | CH₂CF₃ | CH₂CH₂CH₃ |
| A-15 | CH₂CCl₃ | H |
| A-16 | CH₂CCl₃ | CH₃ |
| A-17 | CH₂CCl₃ | CH₂CH₃ |
| A-18 | CH₂CCl₃ | CH₂CH₂CH₃ |
| A-19 | CH(CH₃)₂ | H |
| A-20 | CH(CH₃)₂ | CH₃ |
| A-21 | CH(CH₃)₂ | CH₂CH₃ |
| A-22 | CH(CH₃)₂ | CH₂CH₂CH₃ |
| A-23 | CH₂C(CH₃)₃ | H |
| A-24 | CH₂C(CH₃)₃ | CH₃ |
| A-25 | CH₂C(CH₃)₃ | CH₂CH₃ |
| A-26 | CH₂CH(CH₃)₂ | H |
| A-27 | CH₂CH(CH₃)₂ | CH₃ |
| A-28 | CH₂CH(CH₃)₂ | CH₂CH₃ |
| A-29 | (±) CH(CH₂CH₃)CH₃ | H |
| A-30 | (±) CH(CH₂CH₃)CH₃ | CH₃ |
| A-31 | (±) CH(CH₂CH₃)CH₃ | CH₂CH₃ |
| A-32 | (R) CH(CH₂CH₃)CH₃ | H |
| A-33 | (R) CH(CH₂CH₃)CH₃ | CH₃ |
| A-34 | (R) CH(CH₂CH₃)CH₃ | CH₂CH₃ |
| A-35 | (S) CH(CH₂CH₃)CH₃ | H |
| A-36 | (S) CH(CH₂CH₃)CH₃ | CH₃ |
| A-37 | (S) CH(CH₂CH₃)CH₃ | CH₂CH₃ |
| A-38 | (±) CH(CH₃)-CH(CH₃)₂ | H |
| A-39 | (±) CH(CH₃)-CH(CH₃)₂ | CH₃ |
| A-40 | (±)CH(CH₃)-CH(CH₃)₂ | CH₂CH₃ |
| A-41 | (R) CH(CH₃)-CH(CH₃)₂ | H |
| A-42 | (R) CH(CH₃)-CH(CH₃)₂ | CH₃ |
| A-43 | (R) CH(CH₃)-CH(CH₃)₂ | CH₂CH₃ |
| A-44 | (S) CH(CH₃)-CH(CH₃)₂ | H |
| A-45 | (S) CH(CH₃)-CH(CH₃)₂ | CH₃ |
| A-46 | (S) CH(CH₃)-CH(CH₃)₂ | CH₂CH₃ |
| A-47 | (±) CH(CH₃)-C(CH₃)₃ | H |
| A-48 | (±) CH(CH₃)-C(CH₃)₃ | CH₃ |
| A-49 | (±) CH(CH₃)-C(CH₃)₃ | CH₂CH₃ |
| A-50 | (R) CH(CH₃)-C(CH₃)₃ | H |
| A-51 | (R) CH(CH₃)-C(CH₃)₃ | CH₃ |
| A-52 | (R) CH(CH₃)-C(CH₃)₃ | CH₂CH₃ |
| A-53 | (S) CH(CH₃)-C(CH₃)₃ | H |
| A-54 | (S) CH(CH₃)-C(CH₃)₃ | CH₃ |
| A-55 | (S) CH(CH₃)-C(CH₃)₃ | CH₂CH₃ |
| A-56 | (±) CH(CH₃)-CF₃ | H |
| A-57 | (±) CH(CH₃)-CF₃ | CH₃ |
| A-58 | (±) CH(CH₃)-CF₃ | CH₂CH₃ |
| A-59 | (R) CH(CH₃)-CF₃ | H |
| A-60 | (R) CH(CH₃)-CF₃ | CH₃ |
| A-61 | (R) CH(CH₃)-CF₃ | CH₂CH₃ |
| A-62 | (S) CH(CH₃)-CF₃ | H |
| A-63 | (S) CH(CH₃)-CF₃ | CH₃ |
| A-64 | (S) CH(CH₃)-CF₃ | CH₂CH₃ |
| A-65 | (±) CH(CH₃)-CCl₃ | H |
| A-66 | (±) CH(CH₃)-CCl₃ | CH₃ |
| A-67 | (±) CH(CH₃)-CCl₃ | CH₂CH₃ |
| A-68 | (R) CH(CH₃)-CCl₃ | H |
| A-69 | (R) CH(CH₃)-CCl₃ | CH₃ |
| A-70 | (R) CH(CH₃)-CCl₃ | CH₂CH₃ |
| A-71 | (S) CH(CH₃)-CCl₃ | H |
| A-72 | (S) CH(CH₃)-CCl₃ | CH₃ |
| A-73 | (S) CH(CH₃)-CCl₃ | CH₂CH₃ |
| A-74 | CH₂C(CH₃)=CH₂ | H |
| A-75 | CH₂C(CH₃)=CH₂ | CH₃ |
| A-76 | CH₂C(CH₃)=CH₂ | CH₂CH₃ |
| A-77 | Cyclopentyl | H |
| A-78 | Cyclopentyl | CH₃ |
| A-79 | Cyclopentyl | CH₂CH₃ |
| A-80 | Cyclohexyl | H |
| A-81 | Cyclohexyl | CH₃ |
| A-82 | Cyclohexyl | CH₂CH₃ |
| A-83 | -(CH₂)₄- | |
| A-84 | (±) -(CH₂)₂-CH(CH₃)-CH₂- | |
| A-85 | (R) -(CH₂)₂-CH(GH₃)-CH₂- | |
| A-86 | (S) -(CH₂)₂-CH(CH₃)-CH₂- | |
| A-87 | -(CH₂)₂-CH(OCH₃)-CH₂- | |
| A-88 | -(CH₂)₂-CH(CH₂CH₃)-CH₂- | |
| A-89 | -(CH₂)₂-CH[CH(CH₃)₂]-CH₂- | |
| A-90 | (±)-(CH₂)₃-CH(CH₃)- | |
| A-91 | (±) -CH(CH₃)-(CH₂)₂-CH(CH₃)- | |
| A-92 | -CH₂-CH=CH-CH₂- | |
| A-93 | -(CH₂)₅- | |
| A-94 | (±) -(CH₂)₄-CH(CH₃)- | |
| A-95 | -(CH₂)₂-CH(CH₃)-(CH₂)₂- | |
| A-96 | (±) -(CH₂)₃-CH(CH₃)-CH₂- | |
| A-97 | (R) -(CH₂)₃-CH(CH₃)-CH₂- | |
| A-98 | (S) -(CH₂)₃-CH(CH₃)-CH₂- | |
| A-99 | -(CH₂)₂-C(O[CH₂]₂O)-(CH₂)₂- | |
| A-100 | 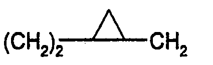 | |
| A-101 | -(CH₂)₂-C(O[CH₂]₃O)-(CH₂)₂- | |
| A-102 | -(CH₂)₂-CH=CH-CH₂- | |

Weiterhin sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

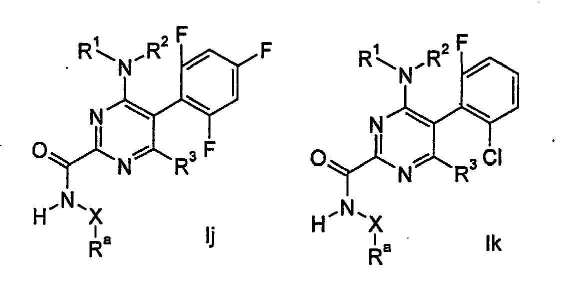

### Tabelle 181

Verbindungen der Formel Ij und Ik in denen NR¹R² 4-Methylpiperidin und R³ Methyl bedeuten und -X-R^{a} für eine Verbindung jeweils einer Zeile der Tabelle B entspricht

### Tabelle 182

Verbindungen der Formel Ij und Ik in denen R¹ CH(CH₃)₂, R² Wasserstoff und R³ Methyl bedeuten und -X-R^{a} für eine Verbindung jeweils einer Zeile der Tabelle B entspricht

### Tabelle 183

Verbindungen der Formel Ij und Ik in denen R¹ CH₂CF₃, R² Wasserstoff und R³ Methyl bedeuten und -X-R^{a} für eine Verbindung jeweils einer Zeile der Tabelle B entspricht

### Tabelle 184

Verbindungen der Formel Ij und Ik in denen R¹(R,S) CH(CH₃)CH₂CH₃, R² Wasserstoff und R³ Methyl bedeuten und -X-R^{a} für eine Verbindung jeweils einer Zeile der Tabelle B entspricht

### Tabelle 185

Verbindungen der Formel Ij und Ik in denen R¹ (R) CH(CH₃)CH₂CH₃, R² Wasserstoff und R³ Methyl bedeuten und -X-R^{a} für eine Verbindung jeweils einer Zeile der Tabelle B entspricht

### Tabelle 186

Verbindungen der Formel Ij und Ik in denen R¹ (S) CH(CH₃)CH₂CH₃, R² Wasserstoff und R³ Methyl bedeuten und -X-R^{a} für eine Verbindung jeweils einer Zeile der Tabelle B entspricht

### Tabelle 187

Verbindungen der Formel Ij und Ik in denen R¹ (R,S) CH(CH₃)CF₃, R² Wasserstoff und R³ Methyl bedeuten und X-R^{a} für eine Verbindung jeweils einer Zeile der Tabelle B entspricht

### Tabelle 188

Verbindungen der Formel Ij und Ik in denen R¹ (R) CH(CH₃)CF₃, R² Wasserstoff und R³ Methyl bedeuten und -X-R^{a} für eine Verbindung jeweils einer Zeile der Tabelle B entspricht

### Tabelle 189

Verbindungen der Formel Ij und Ik in denen R¹ (S) CH(CH₃)CF₃, R² Wasserstoff und R³ Methyl bedeuten und X-R^{a} für eine Verbindung jeweils einer Zeile der Tabelle B entspricht

### Tabelle 190

Verbindungen der Formel Ij und Ik in denen NR¹R² 4-Methylpiperidin und R³ Chlor bedeuten und -X-R^{a} für eine Verbindung jeweils einer Zeile der Tabelle B entspricht

### Tabelle 191

Verbindungen der Formel Ij und Ik in denen R¹ CH(CH₃)₂, R² Wasserstoff und R³ Chlor bedeuten und -X-R^{a} für eine Verbindung jeweils einer Zeile der Tabelle B entspricht

### Tabelle 192

Verbindungen der Formel Ij und Ik in denen R¹ CH₂CF₃, R² Wasserstoff und R³ Chlor bedeuten und -X-R^{a} für eine Verbindung jeweils einer Zeile der Tabelle B entspricht

### Tabelle 193

Verbindungen der Formel Ij und Ik in denen R¹ (R,S) CH(CH₃)CH₂CH₃; R² Wasserstoff und R³ Chlor bedeuten und -_{X}-R^{a} für eine Verbindung jeweils einer Zeile der Tabelle B entspricht

### Tabelle 194

Verbindungen der Formel Ij und Ik in denen R¹ (R) CH(CH₃)CH₂CH₃, R² Wasserstoff und R³ Chlor bedeuten und -X-R^{a} für eine Verbindung jeweils einer Zeile der Tabelle B entspricht

### Tabelle 195

Verbindungen der Formel Ij und Ik in denen R¹ (S) CH(CH₃)CH₂CH₃, R² Wasserstoff und R³ Chlor bedeuten und -X-R^{a} für eine Verbindung jeweils einer Zeile der Tabelle B entspricht

### Tabelle 196

Verbindungen der Formel Ij und Ik in denen R¹ (R,S) CH(CH₃)CF₃, R² Wasserstoff und R³ Chlor bedeuten und -X-R^{a} für eine Verbindung jeweils einer Zeile der Tabelle B entspricht

### Tabelle 197

Verbindungen der Formel Ij und Ik in denen R¹ (R) CH(CH₃)CF₃, R² Wasserstoff und R³ Chlor bedeuten und -X-R^{a} für eine Verbindung jeweils einer Zeile der Tabelle B entspricht

### Tabelle 198

Verbindungen der Formel Ij und Ik in denen R¹ (S) CH(CH₃)CF₃, R² Wasserstoff und R³ Chlor bedeuten und -X-R^{a} für eine Verbindung jeweils einer Zeile der Tabelle B entspricht

### Tabelle 199

Verbindungen der Formel Ij und Ik in denen NR¹R² 4-Methylpiperidin und R³ Methoxy bedeuten und -X-R^{a} für eine Verbindung jeweils einer Zeile der Tabelle B entspricht

### Tabelle 200

Verbindungen der Formel Ij und Ik in denen R¹ CH(CH₃)₂, R² Wasserstoff und R³ Methoxy bedeuten und -X-R^{a} für eine Verbindung jeweils einer Zeile der Tabelle B entspricht

### Tabelle 201

Verbindungen der Formel Ij und Ik in denen R¹ CH₂CF₃, R² Wasserstoff und R³ Methoxy bedeuten und -X-R^{a} für eine Verbindung jeweils einer Zeile der Tabelle B entspricht

### Tabelle 202

Verbindungen der Formel Ij und Ik in denen R¹ (R,S) CH(CH₃)CH₂CH₃, R² Wasserstoff und R³ Methoxy bedeuten und -X-R^{a} für eine Verbindung jeweils einer Zeile der Tabelle B entspricht

### Tabelle 203

Verbindungen der Formel Ij und Ik in denen R¹(R) CH(CH₃)CH₂CH₃, R² Wasserstoff und R³ Methoxy bedeuten und -X-R^{a} für eine Verbindung jeweils einer Zeile der Tabelle B entspricht

### Tabelle 204

Verbindungen der Formel Ij und Ik in denen R¹ (S) CH(CH₃)CH₂CH₃, R² Wasserstoff und R³ Methoxy bedeuten und -X-R^{a} für eine Verbindung jeweils einer Zeile der Tabelle B entspricht

### Tabelle 205

Verbindungen der Formel Ij und Ik in denen R¹ (R,S) CH(CH₃)CF₃, R² Wasserstoff und R³ Methoxy bedeuten und -X-R^{a} für eine Verbindung jeweils einer Zeile der Tabelle B entspricht

### Tabelle 206

Verbindungen der Formel Ij und Ik in denen R¹(R) CH(CH₃)CF₃, R² Wasserstoff und R³ Methoxy bedeuten und -X-R^{a} für eine Verbindung jeweils einer Zeile der Tabelle B entspricht

### Tabelle 207

Verbindungen der Formel Ij und Ik in denen R¹ (S) CH(CH₃)CF₃, R² Wasserstoff und R³ Methoxy bedeuten und -X-R^{a} für eine Verbindung jeweils einer Zeile der Tabelle B entspricht

### Tabelle 208

Verbindungen der Formel Ij und Ik in denen NR¹R² 4-Methylpiperidin und R³ Cyano bedeuten und -X-R^{a} für eine Verbindung jeweils einer Zeile der Tabelle B entspricht

### Tabelle 209

Verbindungen der Formel Ij und Ik in denen R¹ CH(CH₃)₂, R² Wasserstoff und R³ Cyano bedeuten und -X-R^{a} für eine Verbindung jeweils einer Zeile der Tabelle B entspricht

### Tabelle 210

Verbindungen der Formel Ij und Ik in denen R¹ CH₂CF₃, R² Wasserstoff und R³ Cyano bedeuten und -X-R^{a} für eine Verbindung jeweils einer Zeile der Tabelle B entspricht

### Tabelle 211

Verbindungen der Formel Ij und Ik in denen R¹ (R,S) CH(CH₃)CH₂CH₃, R² Wasserstoff und R³ Cyano bedeuten und -X-R^{a} für eine Verbindung jeweils einer Zeile der Tabelle B entspricht

### Tabelle 212

Verbindungen der Formel Ij und Ik in denen R¹ (R) CH(CH₃)CH₂CH₃, R² Wasserstoff und R³ Cyano bedeuten und X-R^{a} für eine Verbindung jeweils einer Zeile der Tabelle B entspricht

### Tabelle 213

Verbindungen der Formel Ij und Ik in denen R¹ (S) CH(CH₃)CH₂CH₃, R² Wasserstoff und R³ Cyano bedeuten und -X-R^{a} für eine Verbindung jeweils einer Zeile der Tabelle B entspricht

### Tabelle 214

Verbindungen der Formel Ij und Ik in denen R¹ (R,S) CH(CH₃)CF₃, R² Wasserstoff und R³ Cyano bedeuten und -X-R^{a} für eine Verbindung jeweils einer Zeile der Tabelle B entspricht

### Tabelle 215

Verbindungen der Formel Ij und Ik in denen R¹ (R) CH(CH₃)CF₃, R² Wasserstoff und R³ Cyano bedeuten und -X-R^{a} für eine Verbindung jeweils einer Zeile der Tabelle B entspricht

### Tabelle 216

Verbindungen der Formel Ij und Ik in denen R¹ (S) CH(CH₃)CF₃, R² Wasserstoff und R³ Cyano bedeuten und X-R^{a} für eine Verbindung jeweils einer Zeile der Tabelle B entspricht

**Tabelle B**

| **Nr.** | **X** | **R^{a}** |
|---|---|---|
| B-1 | -(C=O)- | H |
| B-2 | | CH₃ |
| B-3 | | CH₂CH₃ |
| B-4 | | CH₂CH₂CH₃ |
| B-5 | | CH₂CH(CH₃)₂ |
| B-6 | | CH₂C(CH₃)₃ |
| B-7 | -O- | H |
| B-8 | | CH₃ |
| B-9 | | CH₂CH₃ |
| B-10 | | CH₂CH₂CH₃ |
| B-11 | | CH₂CH(CH₃)₂ |
| B-12 | | CH₂C(CH₃)₃ |
| B-13 | -(C=O)-O- | H |
| B-14 | | CH₃ |
| B-15 | | CH₂CH₃ |
| B-16 | | CH₂CH₂CH₃ |
| B-17 | | CH₂CH(CH₃)₂ |
| B-18 | | CH₂C(CH₃)₃ |
| B-19 | -NH- | H |
| B-20 | | CH₃ |
| B-21 | | CH₂CH₃ |
| B-22 | | CH₂CH₂CH₃ |
| B-23 | | CH₂CH(CH₃)₂ |
| B-24 | | CH₂C(CH₃)₃ |
| B-25 | -(C=O)-NH- | H |
| B-26 | | CH₃ |
| B-27 | | CH₂CH₃ |
| B-28 | | CH₂CH₂CH₃ |
| B-29 | | CH₂CH(CH₃)₂ |
| B-30 | | CH₂C(CH₃)₃ |
| B-31 | direkte Bindung | H |
| B-32 | | CH₃ |
| B-33 | | CH₂CH₃ |
| B-34 | | CH₂CH₂CH₃ |
| B-35 | | CH₂CH(CH₃)₂ |
| B-36 | | CH₂C(CH₃)₃ |
| B-37 | | CH₂OH |
| B-38 | | CH₂CH₂OH |
| B-39 | | CH₂CH₂CH₂OH |
| B-40 | | CH₂CH₂CH₂CH₂OH |
| B-41 | | CH₂OCH₃ |
| B-42 | | CH₂CH₂OCH3 |
| B-43 | | CH₂CH₂CH₂OCH₃ |
| B-44 | | CH₂CH₂CH₂CH₂OCH₃ |
| B-45 | | CH₂OCH₂CH₃. |
| B-46 | | CH₂CH₂OCH₂CH₃ |
| B-47 | | CH₂CH₂CH₂OCH₂CH₃ |
| B-48 | | CH₂NH₂ |
| B-49 | | CH₂CH₂NH₂ |
| B-50 | | CH₂CH₂CH₂NH₂ |
| B-51 | | CH₂NHCH₃ |
| B-52 | | CH₂CH₂NHCH₃ |
| B-53 | | CH₂CH₂CH₂NHCH₃ |
| B-54 | | CH₂NHCH₂CH₃ |
| B-55 | | CH₂CH₂NHCH₂CH₃ |
| B-56 | | CH₂CH₂CH₂NHCH₂CH₃ |
| B-57 | | CH₂N(CH₃)₂ |
| B-58 | | CH₂SH |
| B-59 | | CH₂CH₂SH |
| B-60 | | CH₂CH₂CH₂SH |
| B-61 | | CH₂SCH₃ |
| B-62 | | CH₂CH₂SCH₃ |
| B-63 | | CH₂CH₂CH₂SCH₃ |
| B-64 | | CH₂SCH₂CH₃ |
| B-65 | | CH₂CH₂SCH₂CH₃ |
| B-66 | | CH₂CH₂CH₂SCH₂CH₃ |
| B-67 | | CH(OCH₃)₂ |
| B-68 | | CH₂CH(OCH₃)₂ |
| B-69 | | CH₂CH₂CH(OCH₃)₂ |
| B-70 | | 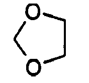 |
| B-71 | | 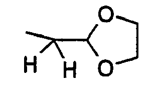 |
| B-72 | | 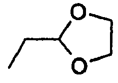 |
| B-73 | | 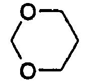 |
| B-74 | | 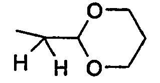 |
| B-75 | | 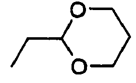 |
| B-76 | | CH=CH₂ |
| B-77 | | CH=CH₂CH₃ |
| B-78 | | CH₂CH=CH₂ |
| B-79 | | CH₂CH₂CH=CH₂ |
| B-80 | | CH₂C≡CH |
| B-81 | | CH₂CH₂C≡CH |

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der Ascomyceten, Deuteromyceten, Oomyceten und Basidiomyceten. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
*Alternaria-*Arten an Gemüse und Obst,
*Bipolaris-* und *Drechslera-*Arten an Getreide, Reis und Rasen,
*Blumeria graminis* (echter Mehltau) an Getreide,
*Botrytis cinerea* (Grauschimmel) an Erdbeeren, Gemüse, Zierpflanzen und Reben, *Erysiphe cichoracearum* und *Sphaerotheca fuliginea* an Kürbisgewächsen,
*Fusarium-* und *Verticillium-*Arten an verschiedenen Pflanzen,
Mycosphaerella-Arten an Getreide, Bananen und Erdnüssen,
*Phytophthora infestans* an-Kartoffeln und Tomaten,
*Plasmopara viticola* an Reben,
*Podosphaera leucotricha* an Äpfeln,
*Pseudocercosporella* herpotrichoides an Weizen und Gerste,
*Pseudoperonospora-*Arten an Hopfen und Gurken,
*Puccinia-*Arten an Getreide,
*Pyricularia oryzae* an Reis,
*Rhizoctonia-*Arten an Baumwolle, Reis und Rasen,
*Septoria tritici* und *Stagonospora nodorum* an Weizen,
*Uncinula necator* an Reben,
*Ustilago-*Arten an Getreide und Zuckerrohr, sowie
*Venturia-*Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:

Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind: 1. Produkte zur Verdünnung in Wasser

### A) Wasserlösliche Konzentrate (SL)

10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.

### B) Dispergierbare Konzentrate (DC)

20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.

### C) Emulgierbare Konzentrate (EC)

15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.

### D) Emulsionen (EW, EO)

40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.

### E) Suspensionen (SC, OD)

20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.

### F) Wasserdispergierbare und wasserlösliche Granulate (WG, SG)

50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.

### G) Wasserdispergierbare und wasserlösliche Pulver (WP, SP)

75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

### H) Stäube (DP)

5 Gew.Teile einer erfindungsgemäßen Verbindung werden fein gemahlen und mit 95 % feinteiligem Kaolin innig vermischt. Man erhält dadurch ein Stäubemittel.

### 1) Granulate (GR, FG, GG, MG)

0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.

### J) ULV- Lösungen (UL)

10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als.95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin,
- Guazatine, Iminoctadine, Spiroxamin, Tridemorph
- Anilinopyrimidine wie Pyrimethanil, Mepanipyrim oder Cyrodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Epoxiconazol, Fenbuconazol, Fluquiconazol, Flusilazol, Flutriafol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel
- Sonstige Fungizide wie Acibenzolar-S-methyl, Benthiavalicarb, Carpropamid, Chlorothalonil, Cyflufenamid, Cymoxanil, Dazomet, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, lprovalicarb, Hexachlorbenzol, Metrafenon, Pencycuron, Propamocarb, Phthalid, Tolclofos-methyl, Quintozene, Zoxamid.
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele

### Beispiel 1: Herstellung von 4-Chloro-6-((S)-2,2,2-trifluoro-1-methyl-ethylamino)-5-(2,4,6-trifluoro-phenyl)-pyrimidin-2-carbonsäureamid [I-5]

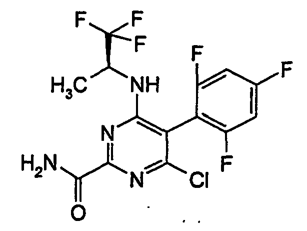

Es wurden 5,0 g 4-Chloro-6-((S)-2,2,2-trifluoro-1-methyl-ethylamino)-5-(2,4,6-trifluorophenyl)-pyrimidin-2-carbonitril (s. WO 03/04993, Seiten 28 und 29) in 5 ml DMSO vorgelegt, dazu wurden 344 mg K₂CO₃, gegeben und auf 10°C abgekühlt. Anschließend wurde 1,4 ml 30%iges H₂O₂ hinzugegeben. Es wurde 5 min. im Eisbad und danach noch 30 min bei Raumtemperatur nachgerührt. Das Reaktionsgemisch wurde in 150 ml Wasser eingetragen. Dabei fiel das Amid aus. Das Amid wurde abfiltriert, gewaschen und im Hochvakuum getrocknet. Es wurden 4,7 g der beige gefärbten Titelverbindung vom Fp. 157-162°C erhalten.

### Beispiel 2: Herstellung von 4-Chloro-6-((S)-2,2,2-trifluoro-1-methyl-ethylamino)-5-(2,4,6-trifluoro-phenyl)-pyrimidin-2-carbonsäure [I-11]

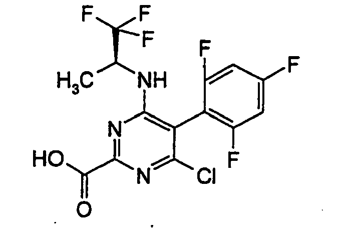

Es wurden 1,5 g 4-Chloro-6-((S)-2,2,2-trifluoro-1-methyl-ethylamino)-5-(2,4,6-trifluorophenyl)-pyrimidin-2-carbonitril (s. WO 03/04993, Seiten 28 und 29) in 5 ml konz. H₂SO₄ gelöst und 20 min bei 110°C gerührt. Das Reaktionsgemisch wurde in 100 ml Eiswasser eingetragen und dabei fiel die Säure aus. Die Säure wurde abfiltriert, mit Wasser gewaschen und im Hochvakuum getrocknet. Man erhielt 1,5 g der gelben Titelverbindung.
¹H-NMR (CDCl₃, ppm): 1.4 (d, CH₃), 4.85 (d, NH), 5.60-5.80 (m, CH), 6.90-7.00 (m, CH), 10.5 (s (breit), OH).

### Beispiel 3: Herstellung von 4-Chloro-6-((S)-2,2,2-trifluoro-1-methyl-ethylamino)-5-(2,4,6-trifluoro-phenyl)-pyrimidin-2-carbonsäure-N-tert.butylamid [I-10]

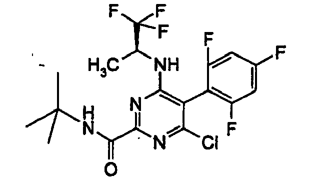

a) Zu 5 ml 40 °C warmen Thionylchlorid wurden 1,5 g der Säure (Beispiel 2) gegeben. Die Reaktionsmischung wurde gerührt bis die Gasentwicklung beendet war. Der Ansatz wurde mit Toluol versetzt und das Lösungsmittel sowie überschüssiges Thionylchlorid komplett abdestilliert. Es wurden 1,6 g eines dunkelgrünen Öls erhalten.
b) Es wurden 38 mg tert-Butylamin und 58 mg Triethylamin in 7 ml THF bei 0°C vorgelegt, dazu wurde 200 mg des zuvor hergestellten Säurechlorid, gelöst in 2 ml THF gegeben. Das Reaktionsgemisch wurde 12 Std. bei Raumtemperatur nachgerührt. Das Reaktionsgemisch wurde einrotiert, in Methyltert.butylether aufgenommen und mit Wasser gewaschen. Die organische Phase wurde abgetrennt, über MgSO₄ getrocknet und eingeengt. Das Rohprodukt wurde mittels präparativer HPLC gereinigt. Es wurden 21 mg der gelben Titelverbindung mit einem Fp. von 49-54°C erhalten.

### Beispiel 4: Herstellung von 4-Chloro-6-((S)-2,2,2-trifluoro-1-methyl-ethylamino)-5-(2,4,6-trifluoro-phenyl)-pyrimidin-2-carbonsäure-N-acetylimid [1-12]

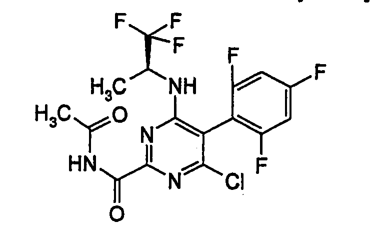

Es wurden 150 mg Amid (Beispiel 1) in 10 ml THF zusammen mit 20 mg Natriumhydrid unter Eisbadkühlung zur Reaktion gebracht und 30 min. nachgerührt. Dazu wurden 35 mg Essigsäurechlorid gelöst in 1 ml THF langsam zugegeben. Es wurde 30 min. bei Raumtemperatur nachgerührt. Anschließend wurde das Reaktionsgemisch mit Eiswasser versetzt und mit Dichlormethan extrahiert. Die verreinigten organischen Phasen wurden über Mg₂SO₄ getrocknet und einrotiert. Es wurden 65 mg der rotbraunen Titelverbindung mit Fp. von 58 bis 65°C erhalten.

### Beispiel 5: Herstellung von 4-Chloro-6-((S)-2,2,2-trifluoro-1-methylethylamino)-5-(2,4,6-trifluorophenyl)-pyrimidin-2-carbonsäureisopropylester- [V-3]

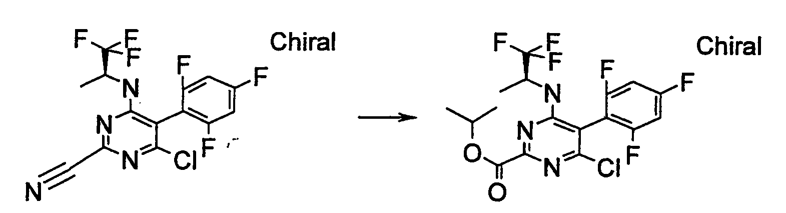

In 210 ml iso-Propanol wurden bei Raumtemperatur 22 g 4-Chloro-6-((S)-2,2,2-trifluoro-1-methylethylamino)-5-(2,4,6-trifluorophenyl)-pyrimidin-2-carbonitril gelöst. In die Lösung wurde während 30 min. HCl-Gas eingeleitet und 96 h bei Rückfluss gerührt. Der Ansatz wurde eingeengt, mit Wasser versetzt, mit Essigester überschichtet und mit Natriumcarbonat alkalisch gestellt. Die Essigester-Phase wurde mit Magnesiumsulfat getrocknet und einrotiert. Man erhielt 21,4g eines farblosen Feststoffes. Ausbeute: 83,8%
Fp.: 146-147°C

Die in den vorstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle I mit physikalischen Daten aufgeführt.

**Tabelle IA**

| | | | | | | |
|---|---|---|---|---|---|---|
| 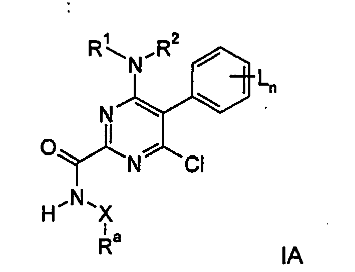 | | | | | | |

| **Nr.** | **R^{a}X** | **R¹** | **R²** | **Lₙ** | **Fp. [°C]** | **¹H-NMR [CD-Cl₃, ppm]** |
|---|---|---|---|---|---|---|
| I-1 | H | (R,S)- CH(CH₃)CH(CH₃)₂ | H | 2,4,6- Trifluor | | 0.80-0.90 (m, 2CH₃), 1.15 (d, CH5), 1.75-1.85 (m, CH), 4.25-4.30 (m, CH), 4.60 (s, NH), 6.75 (s, NH), 6.80-6.90 (m, 2CH), 7.70 (s, NH) |
| I-2 | H | -(CH₂)₂CH(CH₃)(CH₂)₂- | | 2,4,6- Trifluor | 116-128 | |
| I-3 | H | -CH(CH₃)₂ | H | 2-Chlor- 6-fluor | 208-210 | |
| I-4 | NH₂ | -CH(CH₃)₂ | H | 2-Chlor- 6-fluor | 103-108 | |
| I-5 | H | (S) -CH(CH₃)CF₃ | H | 2,4,6- Trifluor | 157-162 | |
| I-6 | NH₂ | (S) -CH(CH₃)CF₃ | H | 2,4,6- Trifluor | 59-65 | |
| I-7 | H | -CH₂C=CH₂ | CH₂C=CH₂ | 2,4,6- Trifluor | 134-141 | |
| I-8 | CH₃ | -CH₂C=CH₂ | CH₂C=CH₂ | 2,4,6- Trifluor | 52-58 | |
| I-9 | N(CH₃)₂ | (S)-CH(CH₃)CF₃ | H | 2,4,6- Trifluor | 39-45 | |
| I-10 | C(CH3)₃ | (S) -CH(CH₃)CF₃ | H | 2,4,6- Trifluor 54 | 49- | |
| I-11 | -OH | (S)-CH(CH₃)CF₃ | H | 2,4,6- Trifluor | | 1.4(d, CH3), 4.85 (d, NH), 5.60-5.80 (m, CH),6.90-7.00 (m,CH), 10.5 (s(breit), OH) |
| I-12 | (C=O)C H₃ | (S) -CH(CH₃)CF₃ | H | 2,4,6- Trifluor | 58-65 | |
| I-13 | H | -CH(CH₃)CF₃ | H | 2,4,6- Trifluor | 222-224 | |
| I-14 | -CH₃ | (S)-CH(CH₃)CF₃ | H | 2,4,6- Trifluor | | 1.25 (d, 3H), 2.9 (d, 3H), 5.25 (m, 2H), 6.75 (m, 2H), 7.8 (s, 1H) |
| I-15 | H | -CH₂-COOH -CH₂-COOH | H | 2,4,6- Trifluor | 227-228 | |
| 1-16 | H | (R)-CH(CH₃)CH(CH₃)₂ | H | 2,4,6- Trifluor | | 0.75 (m, 6 H), 1.1 (m, 3H), 1.75 (m, 1H), 4.2 (m, 1H), 4.4 (m, 1H), 5.8 (s, 1H), 7.25 (m, 2H), 7.4 (m, 1H), 7.6 (s, 1 H) |
| 1-17 | H | -CH(CH₃)₂ | H | 2-Chlor- 4-fluor | 141-150 | |
| I-18 | H | -CH₂-C₆H₅ | H | 2-Chlor- 4-fluor | 48-56 | |
| 1-19 | H | (R)-CH(CH₃)CH(CH₃)₂ | H | 2,4- Difluor | | 0.75 (m, 6H), 1.1 (m, 3H), 1.75 (m, 1H),4.2 (m, 1H), 4.5 (m, 1 H), 5.85 (s, 1 H), 7.1 (m, 2H), 7.3 (m, 1 H), 7.6 (s, 1 H) |
| I-20 | H | -CH₂-C₆H₅ | H | 2,4- Difluor | 53-58 | |
| I-21 | H | -CH(CH₃)₂ | H | 2,4- Difluor | 172-176 | |
| I-22 | H | -(CH₂)₂CH(CH₃)(CH₂)₂- | | 2,4- Difluor | 50-63 | |
| I-23 | H | (S)-CH(CH₃)CH(CH₃)₂ | H | 2,4- Difluor | 99-105 | |
| I-24 | H | (S)-CH(CH₃)CH(CH₃)₂ | H | 2-Chlor- 4-fluor | 79-88 | |
| I-25 | H | -(CH₂)₂CH(CH₃)(CH₂)₂- | | 2,6- Difluor | 173-175 | |
| I-26 | H | -(CH₂)₂CH(CH₃)(CH₂)₂- | | 2-Chlor- 4-fluor | 175-177 | |
| I-27 | H | (S)-CH(CH₃)CF₃ | H | 2, 6- Difluor | 206-208 | |
| I-28 | H | (S)-CH(CH₃)CH(CH₃)₂ | H | 2,6- Difluor | 128-130 | |
| I-29 | H | (S)-CH(CH₃)CF₃ | H | 2-Chlor- 4-fluor | 165-167 | |
| I-30 | (C=O)C H₃ | -(CH₂)₂CH(CH₃)(CH₂)₂- | | 2-Chloro- 4-fluor | | 0.9 (d, 3H), 1.0 (m, 2H), 2.6 (s, 3H), 4.0 (m, 2H), 7.1 (m, 1H), 7.25 (m, 2 H), 10.0 (s, 1H) |
| I-31 | H | (S)-CH(CH₃)CH(CH₃)₂ | H | 2-Chlor- 4- methoxy | 84-88 | |
| I-32 | H | (S)-CH(CH₃)CF₃ | H | 2,4,6- Trifluor | | 1.4 (m, 3 H), 3.9 (s, 3 H), 5.0 (d, 1H), 5.25 (m, 1 H), 6.8 (m, 2H) |
| I-33 | (C=O)C H3 | -(CH₂)₂CH(CH₃)(CH₂)₂- | | 2,6- Difluor | | 0.9 (d, 3H), 1.0 (m, 2H), 1.6 (s, 3H), 2.8 (s, 3H), 4.0 (m, 2H), 7.0 (m, 2H), 7.45 (m, 1H), 10.0 (s, 1 H) |

**Tabelle VA**

| | | | | | | |
|---|---|---|---|---|---|---|
| 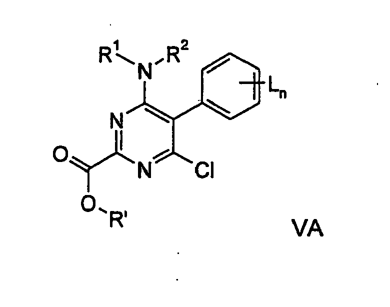 | | | | | | |

| **Nr.** | **R'** | **R¹** | **R²** | **Lₙ** | **Fp.[°C]** | **¹H-NMR [CDCl₃, ppm]** |
|---|---|---|---|---|---|---|
| V-1 | CH₃ | (S)-CH(CH₃)CF₃ | H | 2,4,6- Trifluor | | 1.3(d,3H), 4.0 (s, 3H), 4.6 (d, NH), 5.25 (m, 1H), 6.9 (m, 2H) |
| V-2 | -CH(CH₃)₂ | -CH₂C=CH₂ | - CH₂C=CH₂ | 2,4,6- Trifluor | 53-57 | |
| V-3 | -CH(CH₃)₂ | (S) -CH(CH₃)CF₃ | H | 2,4,6- Trifluor | 146-147 | |
| V-4 | -CH₂CH₃ | (S) -CH(CH₃)CF₃ | H | 2,4,6- Trifluor | | 1.35 (d, 3H), 1.45 (t, 3H), 4.5 (q, 2H), 4.6 (d, NH), 5.3 (m, CH), 6.9 (m, 2H) |
| V-5 | -(CH₂)₂CH₃ | (S) -CH(CH₃)CF₃ | H | 2,4,6- Trifluor | 100-105 | |
| V-6 | -CH(CH₃)₂ | -CH₂CF₃ | H | 2,4,6- Trifluor | 115-118 | |
| V-7 | -(CH₂)₃CH₃ | (S) -CH(CH₃)CF₃ | H | 2,4,6- Trifluor | 71-75 | |
| V-8 | -(CH₂)₄CH₃ | (S) -CH(CH₃)CF₃ | H | 2,4,6- Trifluor | 82-88 | |
| V-9 | -(CH₂)₅CH₃ | (S) -CH(CH₃)CF₃ | H | 2,4,6- Trifluor | 57-60 | |
| V-10 | -CH₂CF₃ | (S)-CH(CH₃)CF₃ | H | 2,4,6- Trifluor | | 1.35 (d, 3H), 4.7 (d, NH), 4.8 (m, 2H),5.3 (m, 1 h), 6.9 (m, 2H) |
| V-11 | (CH₂)₂CH(CH₃)₂ | (S) -CH(CH₃)CF₃ | H | 2,4,6- Trifluor | 90-93 | |
| V-12 | CH₂CH(CH₃)- CH₂CH₃ | (S) -CH(CH₃)CF₃ | H | 2,4,6- 75-Trifluor 81 | | |
| V-13 | -CH(CH₃)- (CH₂)₂CH₃ | (S)-CH(CH₃)CF₃ | | 2,4,6- Trifluor, | | 0.9 (t, 3H), 1.3-1.9 (m, 13H), 4.55 (d, NH), 5.25 (m, 2H), 6.9 (m, 2H) |
| V-14 | -CH(CH₃)₂ | -(CH₂)₂CH(CH₃)(CH₂)₂- | | 2,4,6- Trifluor | | 0.8 (d, 3H), 0.9 (m, 2H), 1.4 (d, 6H), 1.5 (m, 3H), 2.7 (t, 2H), 3.9 (d, 2H), 5.2 (m, CH), 6.7 (m, 2H) |
| V-15 | -CH(CH₃)- CH(CH₃)₂ | (S)-CH(CH₃)CF₃ | H | 2,4,6- Trifluor | | 1.0 (m, 6H), 1.4 (m 6H), 2.0 (m, CH), 4.6 (m, NH), 5.0 (m, CH), 5.3 (m, CH), 6.9 (m, 2H) |
| V-16 | -CH₂C=CH₂ | (S) -CH(CH₃)CF₃ | H | 2,4,6- Trifluor | | 1.35 (d, 3H), 4.8 (d, 2H), 4.95 (d, 1H), 6.3 (m, 2H), 5.45 (d, 1H), 6.1 (m, 1H), 6.9 (m, 1 H) |
| V-17 | -(CH₂)₂NH₂ | (S)-CH(CH₃)CF₃ | H | 2,4,6- Trifluor | | 1.0 (m, 6H), 1.4 (m 6H), 2.0(m, CH), 4.6 (m, NH), 5.0 (m, CH), 5.3 (m, CH), 6.9 (m, 2H) |
| V-18 | Cyclohexyl | (S) -CH(CH₃)CF₃ | H | 2,4,6- Trifluor, | | 1.35(d,3H),4.8 (d, 2H), 4.95 (d, 1H), 6.3(m, 2H), 5.45 (d, |
| | | | | | | 1H), 6.1 (m, 1 H), 6.9 (m, 1 H) |
| V-19 | -CH₂CH(CH₃)₂ | (S) -CH(CH₃)CF₃ | H | 2,4,6- Trifluor | 97-102 | |
| V-20 | - CH(CH₃)CH₂CH₃ | (S) -CH(CH₃)CF₃- | H | 2,4,6- Trifluor | 145-146 | |
| V-21 | -CH₂C₆H₅ | (S) -CH(CH₃)CF₃ | H | 2,4,6- Trifluor | | 1.4 (d, 3H), 4.55 (m, 3H), 5.25 (m, 1H), 6.9 (m, 2H), 7.3-7.5 (m, 5H) |
| V-22 | Cyclopentyl | (S) -CH(CH₃)CF₃ | H | 2,4,6- Trifluor | 114-115 | |
| V-23 | Cyclobutyl | (S) -CH(CH₃)CF₃ | H | 2,4,6- Trifluor | 127-129 | |

Beispiele für die Wirkung gegen Schadpilze

Die fungizide Wirkung der Verbindungen der Formel I ließ sich durch die folgenden Versuche zeigen:

Die Wirkstoffe wurden getrennt als Stammlösung formuliert mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf Basis ethoxylierter Alkylphenole) zugesetzt. Die Stammlösungen der Wirkstoffe wurden entsprechend der angegebenen Konzentration mit Wasser verdünnt.

### Anwendungsbeispiele

### 1. Wirkung gegen die Krautfäule an Tomaten verursacht durch Phytophthora infestans bei protektiver Behandlung

Blätter von Topfpflanzen der Sorte "goldene Prinzessin" wurden mit einer wässriger Suspension in der unten angegebenen Wirkstoffkonzentration bis zur Tropfnässe besprüht. Am folgenden Tag wurden die Blätter mit einer wässrigen Sporangienaufschwemmung von *Phytophthora infestans* infiziert. Anschließend wurden die Pflanzen in einer wasserdampf-gesättigten Kammer bei Temperaturen zwischen 18 und 20°C aufgestellt. Nach 6 Tagen hatte sich die Krautfäule auf den unbehandelten, jedoch infizierten Kontrollpflanzen so stark entwickelt, dass der Befall visuell in % ermittelt werden konnte.

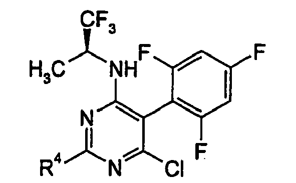

| **Nr.** | **R⁴** | **Dokument** | **Befall bei 250 ppm a.i. (%Blattfläche)** |
|---|---|---|---|
| I-5 | -(C=O)NH₂ | erfindungsgemäß | 0 |
| V-3 | -(C=O)-O-CH(CH)₃ | erfindungsgemäß | 5 |
| V1 | -(C=NOCH₃)NH₂ | WO 03/043993 | 80 |
| | | | |
| | unbehandelt | | 80 |

### 2. Dauerwirksamkeit gegen die Dürrfleckenkrankheit der Tomate verursacht durch Alternaria solani bei protektiver Behandlung

Blätter von Topfpflanzen der Sorte "Goldene Prinzessin" wurden mit einer wässriger Suspension in der unten angegebenen Wirkstoffkonzentration bis zur Tropfnässe besprüht. Um die Dauerwirkung zu testen wurden erst sieben Tage später die Blätter mit einer wässrigen Sporenaufschwemmung von *Alternaria solani* in 2 % Biomalzlösung mit einer Dichte von 0.17 x 10⁶ Sporen/ml infiziert. Anschließend wurden die Pflanzen in einer wasserdampf-gesättigten Kammer bei Temperaturen zwischen 20 und 22°C aufgestellt. Nach weiteren 5 Tagen hatte sich die Krankheit auf den unbehandelten, jedoch infizierten Kontrollpflanzen so stark entwickelt, dass der Befall visuell in % ermittelt werden konnte.

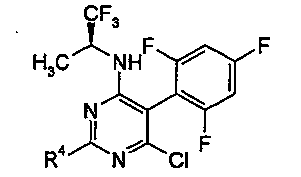

| **Nr.** | **R⁴** | **Dokument** | **Befall bei 16 ppm a.i. (%Blattfläche)** |
|---|---|---|---|
| I-5 | (C=O)NH₂ | erfindungsgemäß | 30 |
| | | | |
| V1 | (C=NOCH₃)NH₂ | WO 03/043993 | 67 |
| | | | |
| | unbehandelt | | 90 |

### 3. Wirksamkeit gegen den Grauschimmel an Paprikablättern verursacht durch Botrytis cinerea bei protektiver Anwendung

Paprikasämlinge der Sorte "Neusiedler Ideal Elite" wurden, nachdem sich 2 - 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1.7 x 10⁶ Sporen/ml in einer 2 %igen wässrigen Biomalzlösung enthielt, inokuliert. Anschließend wurden die Versuchspflanzen in eine Klimakammer mit 22 bis 24°C, Dunkelheit und hoher Luftfeuchtigkeit gestellt. Nach 5 Tagen konnte das Ausmaß des Pilzbefalls auf den Blättern visuell in % ermittelt werden.

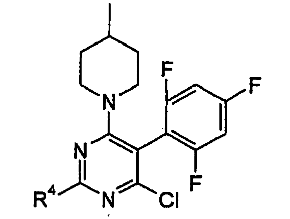

| **Nr.** | **R⁴** | **Dokument** | **Befall bei 16 ppm a.i. (%Blattfläche)** |
|---|---|---|---|
| 1-2 | -(C=O)NH₂ | erfindungsgemäß | 15 |
| | | | |
| V2 | -(C=NOCH₃)NH₂ | Beispiel I-186 aus WO 03/043993 | 90 |
| | | | |
| | unbehandelt | | 100 |

## Patentansprüche

1. 2-Substituierte Pyrimidine der Formel I 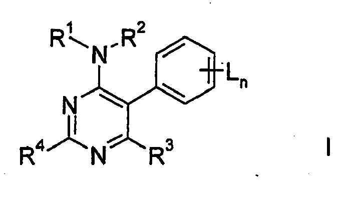 in der der Index und die Substituenten die folgende Bedeutung haben:
n eine ganze Zahl von 1 bis 5;
L Halogen, Cyano, Cyanato (OCN), C₁-C₈-Alkyl, C₂-C₈-Alkenyl, C₂-C₈-Alkinyl, C₁-C₆-Alkoxy, C₂-C₈-Alkenyloxy, C₂-C₈-Alkinyloxy, C₃-C₆-Cycloalkyl, C₄-C₆-Cycloalkenyl, C₃-C₆-Cycloalkyloxy, C₄-C₆-Cycloalkenyloxy, Nitro, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A,
m 0, 1 oder 2;
A, A', A" unabhängig voneinander Wasserstoff, C₁-C₆-Alkyl, C₂-C₆-Alkenyl, C₂-C₆-Alkinyl, C₃-C₈-Cycloalkyl, C₃-C₈-Cycloalkenyl, Phenyl, wobei die organischen Reste partiell oder vollständig halogeniert sein können oder durch Nitro, Cyanato, Cyano oder C₁-C₄-Alkoxy substituiert sein können; oder A und A' zusammen mit den Atomen an die sie gebunden sind für einen fünf- bis sechsgliedrigen gesättigten, partiell ungesättigten oder aromatischen Heterocyclus, enthaltend ein bis vier Heteroatome aus der Gruppe O, N oder S, stehen;
wobei die aliphatischen Gruppen der Restedefinitionen von L ihrerseits partiell oder vollständig halogeniert sein oder eine bis vier Gruppen R^{U} tragen können:
R^{u} Cyano, C₁-C₆-Alkoxy, C₃-C₆-Cycloalkyl, C₂-C₈-Alkenyloxy, C₂-C₈-Alkinyloxy, C₄-C₆-Cycloalkenyl, C₃-C₆-Cycloalkyloxy, C₄-C₆-Cycloalkenyloxy -C(=O)-A, -C(=O)-O-A, -C(=O)-N(A')A, C(A')(=N-OA), N(A')A, N(A')-C(=O)-A, N(A")-C(=O)-N(A')A, S(=O)ₘ A, S(=O)ₘ-O-A oder S(=O)ₘ-N(A')A;
R¹, R² unabhängig voneinander C₁-C₆-Alkyl, C₂-C₆-Alkenyl, C₂-C₆-Alkinyl, C₃-C₆-Cycloalkyl, C₃-C₆-Halogencycloalkyl, wobei die aliphatischen Gruppen der Restedefinitionen von R¹ und R² ihrerseits partiell oder vollständig halogeniert sein oder eine bis vier Gruppen R^{v} tragen können:
R^{v} Cyano, C₃-C₆-Cycloalkyl, C₄-C₆-Cycloalkenyl, Hydroxy, C₁-C₆-Alkoxy, C₂-C₈-Alkenyloxy, C₂-C₈-Alkinyloxy, C₃-C₆-Cycloalkyloxy, C₄-C₆-Cycloalkenyloxy, C₁-C₆-Alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A oder Phenyl, wobei der Phenylteil ein bis drei Reste ausgewählt aus der Gruppe: Halogen, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy, Cyano, Nitro, -C(=O)-A, -C(=O)-O-A, -C(=O)-N(A')A, C(A')(=N-OA), N(A')A tragen kann;
R² kann zusätzlich Wasserstoff bedeuten;
R¹ und R² können auch zusammen mit dem Stickstoffatom, an das sie gebunden sind, einen gesättigten oder ungesättigten fünf- oder sechsgliedrigen Ring bilden, der durch eine Ether -(-O-), Carbonyl -(C=O)-, Thio -(-S-), Sulfoxyl -(-S[=O]-) oder Sulfenyl -(-SO₂-) oder eine weitere Amino -(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
R³ Halogen, Cyano, C₁-C₄-Alkyl, C₂-C₄-Alkenyl, C₂-C₄-Alkinyl, C₃-C₆-Cycloalkyl, C₁-C₄-Alkoxy, C₃-C₄-Alkenyloxy, C₃-C₄-Alkinyloxy, C₁-C₆-Alkylthio, Di-(C₁-C₆-alkyl)amino oder C₁-C₆-Alkylamino, wobei die Alkyl, Alkenyl und Alkinylreste von R³ durch Halogen, Cyano, Nitro, C₁-C₂-Alkoxy oder C₁-C₄-Alkoxycarbonyl substituiert sein können;
R⁴ einer der Formeln 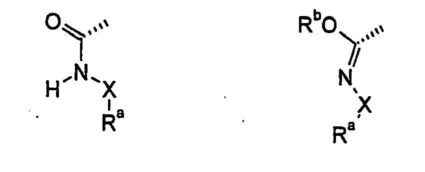 entspricht, in denen
X eine direkte Bindung, -(C=O)-, -(C=O)-NH-, -(C=O)-O-, -O-, -NR^{c}-, -CH₂O-(C=O)-, -C=C-(C=O)-, wobei der jeweils linke Molekülteil an das Stickstoffatom gebunden ist;
R^{a} Wasserstoff, C₁-C₆-Alkyl, C₂-C₈-Alkenyl, C₂-C₈-Alkinyl oder Benzyl;
R^{b} Wasserstoff, C₁-C₆-Alkyl, C₂-C₆-Alkenyl oder C₂-C₈-Alkinyl;
R^{c} Wasserstoff, C₁-C₆-Alkyl, C₂-C₆-Alkenyl, C₂-C₈-Alkinyl, Benzyl oder C₁-C₆-Acyl
bedeuten,
wobei die aliphatischen, alicyclischen oder aromatischen Gruppen der Restedefinitionen von R^{a}, R^{b} und/oder R^{c} ihrerseits eine bis vier Gruppen R^{w} tragen können:
R^{w} Halogen, Cyano, OR^{x}, NHR^{x}, SR^{x}, C₁-C₆-Alkyl, C₂-C₆-Alkenyl, C₂-C₆-Alkinyl, C₁-C₆-Alkoxy, C₁-C₄-Alkoxycarbonyl, C₁-C₄-Acylamino, [1,3]Dioxolane-C₁-C₄-alkyl, [1,3]Dioxane-C₁-C₄-alkyl, wobei
R^{x} Wasserstoff, C₁-C₆-Alkyl, C₂-C₈-Alkenyl, C₂-C₈-Alkinyl oder Benzyl bedeutet.

2. 2-Substituierte Pyrimidine der Formel I nach Anspruch 1, in der der Index und die Substituenten die folgende Bedeutung haben:
n eine ganze Zahl von 1 bis 3, wobei mindestens ein Substituent L in ortho-Stellung am Phenylring sitzt;
L Halogen, Cyano, Methyl, Methoxy, -C(=O)-O-A, -C(=O)-N(A')A, C(A')(=N-OA), N(A')A, N(A')-C(=O)-A,
A,A' unabhängig voneinander Wasserstoff; C₁-C₆-Alkyl, C₂-C₆-Alkenyl, C₂-C₆-Alkinyl, Phenyl, wobei die organischen Reste partiell oder vollständig halogeniert sein können oder durch C₁-C₄-Alkoxy substituiert sein können; oder A und A' zusammen mit den Atomen an die sie gebunden sind für einen fünf- bis sechsgliedrigen gesättigten Heterocyclus, enthaltend ein oder zwei Heteroatome aus der Gruppe O, N oder S, stehen;
wobei die aliphatischen Gruppen der Restedefinitionen von L ihrerseits partiell oder vollständig halogeniert sein können;
R¹,R² unabhängig voneinander C₁-C₆-Alkyl, C₂-C₆-Alkenyl, C₂-C₆-Alkinyl, C₁-C₆-Halogenalkyl, C₂-C₆-Halogenalkenyl oder C₂-C₆-Haiogenatkinyl;
R² kann zusätzlich Wasserstoff bedeuten;
R¹ und R² können auch zusammen mit dem Stickstoffatom, an das sie gebunden sind, einen gesättigten oder ungesättigten fünf- oder sechsgliedrigen Ring bilden, der durch eine Ether-(-O-) oder eine weitere Amino-(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
R³ Halogen, Cyano, C₁-C₄-Alkyl, C₁-C₄-Alkoxy oder C₁-C₄-Haloalkyl;
R⁴ einer der Formeln 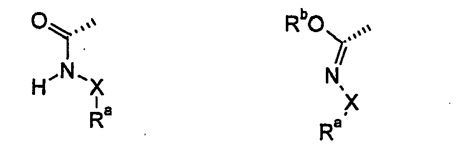 entspricht, in denen
X eine direkte Bindung, -(C=O)-, -(C=O)-NH-, -(C=O)-O-, -O-, -NR^{c}-, wobei der jeweils linke Molekülteil an das Stickstoffatom gebunden ist;
R^{a} Wasserstoff, Methyl, Allyl oder Propargyl;
R^{b} Wasserstoff, C₁-C₄-Alkyl, Allyl oder Propargyl;
R^{c} Wasserstoff, Methyl oder C₁-C₄-Acyl
bedeuten,
wobei die aliphatischen Gruppen der Restedefinitionen von R^{a}, R^{b} und/oder R^{c} ihrerseits eine oder zwei Gruppen R^{w} tragen können:
R^{w} Halogen, OR^{x}, NHR^{x}, C₁-C₆-Alkyl, C₁-C₄-Alkoxycarbonyl, C₁-C₄-Acylamino, [1,3]Dioxolane-C₁-C₄-alkyl, [1,3]Dioxane-C₁-C₄-alkyl, wobei
R^{x} Wasserstoff, Methyl, Allyl oder Propargyl bedeutet.

3. 2-Substituierte Pyrimidine nach Anspruch 1, wobei R³ Chlor, Cyano, Methyl oder Methoxy bedeutet.

4. 2-Substituierte Pyrimidine nach Anspruch 1, wobei wobei R⁴ einer Formel 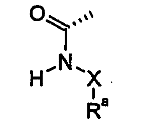 entspricht, wobei X für eine direkte Bindung, -O- oder -(C=O)-O- steht, und R^{a} Wasserstoff oder C₁-C₆-Alkyl bedeutet.

5. 2-Substituierte Pyrimidine nach einem der Ansprüche 1 bis 6, in der die durch Lₙ substituierte Phenylgruppe für die Gruppe B 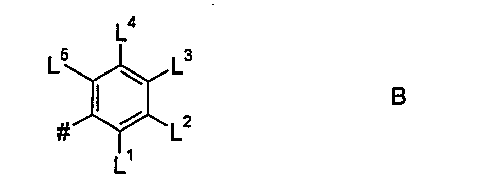 steht, worin # die Verknüpfungsstelle mit dem Pyrimidin-Gerüst ist und
L¹ Fluor, Chlor, CH₃ oder CF₃;
L²,L⁴ unabhängig voneinander Wasserstoff, CH₃ oder Fluor;
L³ Wasserstoff, Fluor, Chlor, Cyano, CH₃, SCH₃, OCH₃, SO₂CH₃, NH-C(=O)CH₃, N(CH₃)-C(=O)CH₃ oder COOCH₃ und
L⁵ Wasserstoff, Fluor, Chlor oder CH₃ bedeuten.

6. Verfahren zur Herstellung der Verbindungen IA, durch Hydrolyse 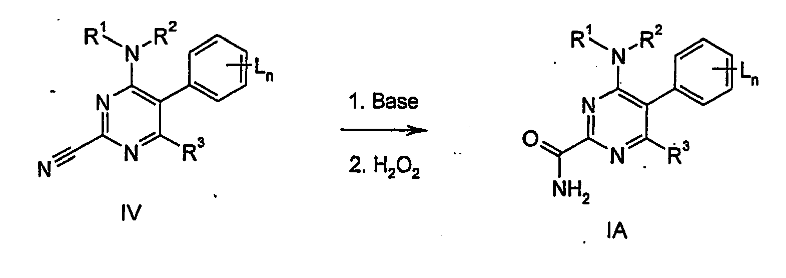 der Nitrile der Formel IV, wobei die Substituenten R¹, R², R³ und L sowie der Index n die in Anspruch 1 angegebene Bedeutung haben, **dadurch gekennzeichnet, dass** in Gegenwart einer Base und Wasserstoffperoxid hydrolysiert wird.

7. Verfahren zur Herstellung der erfindungsgemäßen Verbindungen IA' und IC, wobei die Substituenten Lₙ, R¹, R², R³ X, R^{a} und R^{b} die in Anspruch 1 gegebene Bedeutung haben, ausgehend von Nitrilen der Formel IV, 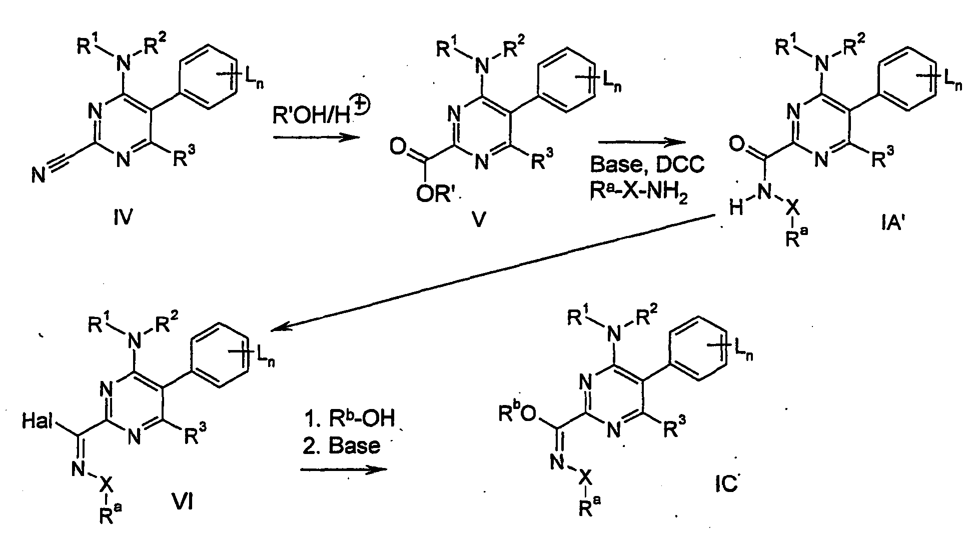 die mit Alkoholen der Formel R'OH, wobei R' C₁-C₈-Alkyl, C₂-C₈-Alkenyl, C₂-C₈-Alkinyl oder C₃-C₆-Cycloalkyl, wobei die Reste Alkyl, Alkenyl und Alkinyl partiell oder vollständig halogeniert sein können und eine bis drei Gruppen R^{V} tragen können, bedeutet, zu den Estern der Formel V, anschließend mit Aminen R^{a}-X-NH₂ unter Zusatz wasserentziehender Mittel zu den Amiden IA' und weiterhin in Gegenwart von Tetrahalogenkohlenstoff und Triarylphosphin zu den Iminhalogeniden der Formel VI und schließlich mit Alkoholen der Formel R^{b}OH und Basen zu den Iminoethern der Formel IC umgesetzt werden.

8. Ester der Formel V 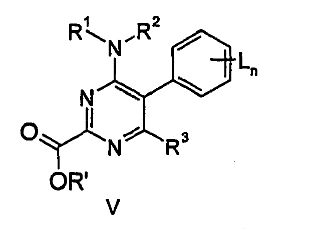 in der die Substituenten R¹, R², R³ und Lₙ die in Anspruch 1 gegebene Bedeutung haben und R' C₁-C₈-Alkyl, C₂-C₈-Alkenyl, C₂-C₈-Alkinyl oder C₃-C₆-Cyclooalkyl, wobei die Reste Alkyl, Alkenyl und Alkinyl partiell oder vollständig halogeniert sein können und eine bis drei Gruppen R^{v} tragen können, bedeutet.

9. Ester V gemäß Anspruch 8, wobei R' Isopropyl bedeutet.

10. Iminhalogenide der Formel VI 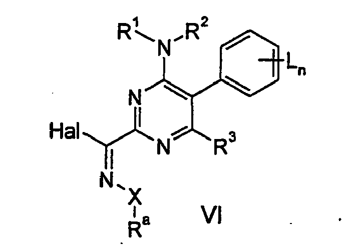 wobei die Substituenten Lₙ, R¹, R², R³, X und R³, X und R² die in Anspruch 1 gegebene Bedeutung haben und Hal für Fluor, Chlor, Brom oder lod steht.

11. Pestizides Mittel, enthaltend einen festen oder flüssigen Trägerstoff und eine Verbindung der Formel I gemäß Anspruch 1.

12. Pestizides Mittel, enthaltend einen festen oder flüssigen Trägerstoff und eine Verbindung der Formel V gemäß einem der Ansprüche 8 oder 9.

13. Verfahren zur Bekämpfung von pflanzenpathogenen Schadpilzen, **dadurch gekennzeichnet, dass** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

14. Verfahren zur Bekämpfung von pflanzenpathogen Schadpilzen, **dadurch gekennzeichnet, dass** man die Pilze oder die von Pilzbefall zu schützenden Materialien, Pflanzen, den Boden oder Saatgüter mit einer wirksamen Menge einer Verbindung der Formel V gemäß einem der Ansprüche 8 oder 9 behandelt.

## Claims

1. A 2-substituted pyrimidine of the formula I 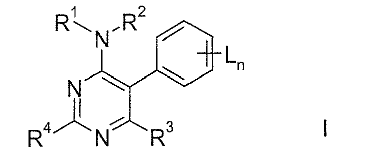 in which the index and the substituents are as defined below:
n is an integer from 1 to 5;
L is halogen, cyano, cyanato (OCN), C₁-C₈-alkyl, C₂-C₈-alkenyl, C₂-C₈-alkynyl, C₁-C₆-alkoxy, C₂-C₈-alkenyloxy, C₂-C₈-alkynyloxy, C₃-C₆-cycloalkyl, C₄-C₆-cycloalkenyl, C₃-C₆-cycoalkyloxy, C₄-C₆-cycloalkenyloxy, nitro, -C(=O)-A, -C(=O)-O-A,-C(=O)-N(A')A, C(A')(=N-OA), N(A')A, N(A')-C(=O)-A, N(A'')-C(=O)-N(A')A, S(=O)ₘ-A, S(=O)ₘ-O-A or S(=O)ₘ-N(A')A,
m is 0, 1 or 2;
A, A', A'' independently of one another are hydrogen, C₁-C₆-alkyl, C₂-C₆-alkenyl, C₂-C₆-alkynyl, C₃-C₈-cycloalkyl, C₃-C₈-cycloalkenyl, phenyl, where the organic radicals may be partially or fully halogenated or may be substituted by nitro, cyanato, cyano or C₁-C₄-alkoxy; or A and A' together with the atoms to which they are attached are a five- or six-membered saturated, partially unsaturated or aromatic heterocycle which contains one to four heteroatoms from the group consisting of O, N and S;
where the aliphatic groups of the radical definitions of L for their part may be partially or fully halogenated or may carry one to four groups R^{u};
R^{u} is cyano, C₁-C₆-alkoxy, C₃-C₆-cycloalkyl, C₂-C₈-alkenyloxy, C₂-C₈-alkynyloxy, C₄-C₆-cycloalkenyl, C₃-C₆-cycloalkyloxy, C₄-C₆-cycloalkenyloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A;
R¹,R² independently of one another are C₁-C₆-alkyl, C₂-C₆-alkenyl, C₂-C₆-alkynyl, C₃-C₆-cycloalkyl, C₃-C₆-halocycloalkyl, where the aliphatic group of the radical definitions of R¹ and R² for their part may be partially or fully halogenated or may carry one to four groups R^{v}:
R^{v} is cyano, C₃-C₆-cycloalkyl, C₄-C₆-cycloalkenyl, hydroxyl, C₁-C₆-alkoxy, C₂-C₈-alkenyloxy, C₂-C₈-alkynyloxy, C₃-C₆-cycloalkyloxy, C₄-C₆-cycloalkenyloxy, C₁-C₆-alkylthio, -C(=O)-A, -C(=O)-O-A, -C(=O)-N (A') A, C(A') (=N-OA), N(A')A, N(A')-C(=O)-A, N(A")-C(=O)-N(A')A, S(=O)ₘ-A, S(=O)ₘ-O-A or S(=O)ₘ-N(A')A or phenyl, where the phenyl moiety may carry one to three radicals selected from the group consisting of: halogen, C₁-C₆-alkyl, C₂-C₆-alkenyl, C₂-C₆-alkynyl, C₃-C₆-cycloalkyl, C₁-C₆-haloalkyl, C₁-C₆-alkoxy, cyano, nitro, -C(=O)-A,-C(=O)-O-A, -C(=O)-N(A')A, C(A')(-N-OA), N(A')A;
R² may additionally be hydrogen;
R¹ and R² may also, together with the nitrogen atom to which they are attached, form a saturated or unsaturated five- or six-membered ring which may be interrupted by an ether (-O-), carbonyl (C=O), thio (-S-), sulfoxyl (-S[=O]-) or sulfenyl (-SO₂-) or a further amino (-N(R^{a}) group, where R^{a} is hydrogen or C₁-C₆-alkyl, and/or may contain one or more substituents from the group consisting of halogen, C₁-C₆-alkyl, C₁-C₆-haloalkyl and oxy-C₁-C₃-alkyleneoxy;
R³ is halogen, cyano, C₁-C₄-alkyl, C₂-C₄-alkenyl, C₂-C₄-alkynyl, C₃-C₆-cycloalkyl, C₁-C₄-alkoxy, C₃-C₄-alkenyloxy, C₃-C₄-alkynyloxy, C₁-C₆-alkylthio, di-(C₁-C₆-alkyl) amino or C₁-C₆-alkylamino, where the alkyl, alkenyl and alkynyl radicals of R³ may be substituted by halogen, cyano, nitro, C₁-C₂-alkoxy or C₁-C₄-alkoxycarbonyl ;
R⁴ corresponds to one of the formulae 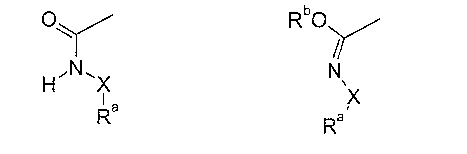 where
X is a direct bond, - (C=O) -, -(C=O)-NH-,-(C=O)-O-, -O-, -NR^{c}-, -CH₂O-(C=O)-, -C=C-(C=O)-, where in each case the left moiety is attached to the nitrogen atom;
R^{a} is hydrogen, C₁-C₆-alkyl, C₂-C₈-alkenyl, C₂-C₈-alkynyl or benzyl;
R^{b} is hydrogen, C₁-C₆-alkyl, C₂-C₆-alkenyl or C₂-C₈-alkynyl;
R^{c} is hydrogen, C₁-C₆-alkyl, C₂-C₆-alkenyl, C₂-C₈-alkynyl, benzyl or C₁-C₆-acyl, where the aliphatic, alicyclic or aromatic groups of the radical definitions of R^{a}, R^{b} and/or R^{c} for their part may carry one to four groups R^{w}:
R^{w} is halogen, cyano, OR^{x}, NHR^{x}, SR^{x}, C₁-C₆-alkyl, C₂-C₆-alkenyl, C₂-C₆-alkynyl, C₁-C₆-alkoxy, C₁-C₄-alkoxycarbonyl, C₁-C₄-acylamino, [1,3]dioxolane-C₁-C₄-alkyl, [1,3]dioxane-C₁-C₄-alkyl, where
R^{x} is hydrogen, C₁-C₆-alkyl, C₂-C₈-alkenyl, C₂-C₈-alkynyl or benzyl.

2. The 2-substituted pyrimidine of the formula 1 according to claim 1 in which the index and the substituents are as defined below:
n is an integer from 1 to 3, where at least one substituent L is located in the ortho-position on the phenyl ring;
L is halogen, cyano, methyl, methoxy, -C(=O)-O-A, -C(=O)-N(A')A, C(A')(=N-OA), N(A')A, N(A')-C(=O)-A,
A,A' independently of one another are hydrogen, C₁-C₆-alkyl, C₂-C₆-alkenyl, C₂-C₆-alkynyl, phenyl, where the organic radicals may be partially or fully halogenated or may be substituted by C₁-C₄-alkoxy; or A and A' together with the atoms to which they are attached are a five- or six-membered saturated heterocycle which contains one or two heteroatoms from the group consisting of O, N and S;
where the aliphatic groups of the radical definitions of L for their part may be partially or fully halogenated;
R¹,R² independently of one another are C₁-C₆-alkyl, C₂-C₆-alkenyl, C₂-C₆-alkynyl, C₁-C₆-haloalkyl, C₂-C₆-haloalkenyl or C₂-C₆-haloalkynyl;
R² may additionally be hydrogen;
R¹ and R² may also, together with the nitrogen atom to which they are attached, form a saturated or unsaturated five- or six-membered ring which may be interrupted by an ether (-O-) or a further amino (-N(R^{a}) group, where R^{a} is hydrogen or C₁-C₆-alkyl, and/or may contain one or more substituents from the group consisting of halogen, C₁-C₆-alkyl, C₁-C₆-haloalkyl and oxy-C₁-C₃-alkyleneoxy;
R³ is halogen, cyano, C₁-C₄-alkyl, C₁-C₄-alkoxy or C₁-C₄-haloalkyl;
R⁴ corresponds to one of the formulae 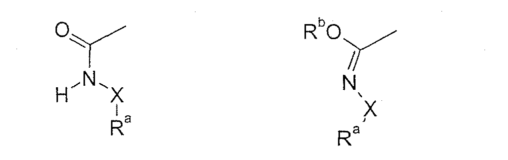 where
X is a direct bond, - (C=O)-, -(C=O)-NH-,-(C=O)-O-, -O-, -NR^{c}-, where in each case the left moiety is attached to the nitrogen atom;
R^{a} is hydrogen, methyl, allyl or propargyl;
R^{b} is hydrogen, C₁-C₄-alkyl, allyl or propargyl;
R^{c} is hydrogen, methyl or C₁-C₄-acyl,
where the aliphatic groups of the radical definitions of R^{a}, R^{b} and/or R^{c} for their part may carry one or two groups R^{w}:
R^{w} is halogen, OR^{x}, NHR^{x}, C₁-C₆-alkyl, C₁-C₄-alkoxycarbonyl, C₁-C₄-acylamino, [1,3]dioxolane-C₁-C₄-alkyl , [1,3]dioxane-C₁-C₄-alkyl, where
R^{x} is hydrogen, methyl, allyl or propargyl.

3. The 2-substituted pyrimidine according to claim 1
in which R³ is chlorine, cyano, methyl or methoxy.

4. The 2-substituted pyrimidine according to claim 1
in which R⁴ corresponds to a formula 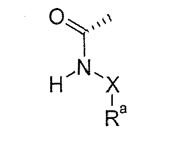 where X is a direct bond, -O- or -(C=O)-O-, and R^{a} is hydrogen or C₁-C₆-alkyl.

5. The 2-substituted pyrimidine according to any of claims 1 to 6 in which the phenyl group substituted by Lₙ is the group B 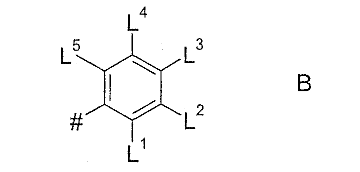 where # is the point of attachment to the pyrimidine skeleton and
L¹ is fluorine, chlorine, CH₃ or CF₃;
L², L⁴ independently of one another are hydrogen, CH₃ or fluorine;
L³ is hydrogen, fluorine, chlorine, cyano, CH₃, SCH₃, OCH₃, SO₂CH₃, NH-C(=O)CH₃, N(CH₃)-C(=O)CH₃ or COOCH₃ and
L⁵ is hydrogen, fluorine, chlorine or CH₃.

6. A process for preparing the compounds IA by hydrolysis 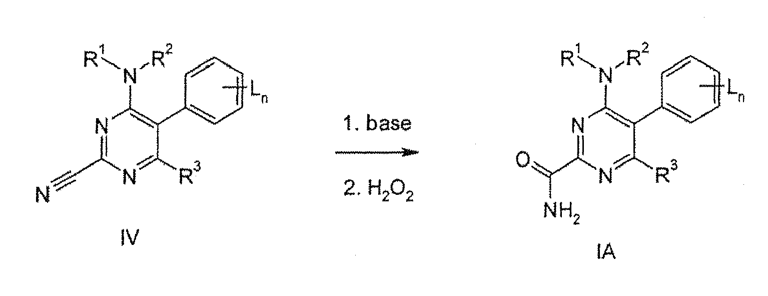 of the nitriles of the formula IV, where the substituents R¹, R², R³ and L and the index n are as defined in claim 1, which comprises carrying out the hydrolysis in the presence of a base and of hydrogen peroxide.

7. A process for preparing the compounds IA' and IC according to the invention where the substituents Lₙ, R¹, R², R³, X, R^{a} and R^{b} are as defined in claim 1, which process uses nitriles of the formula IV 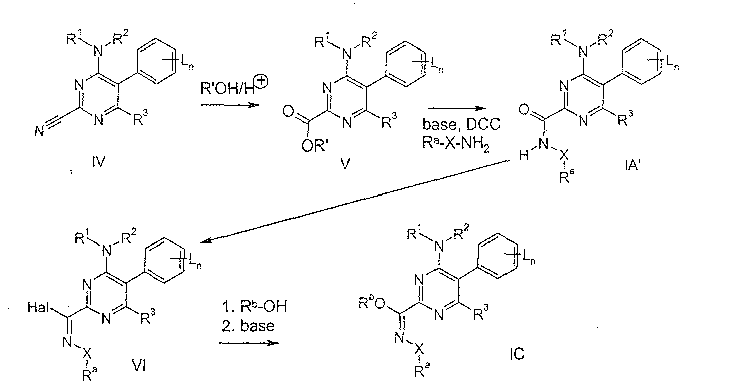 which are converted with alcohols of the formula R'OH, where R' is C₁-C₈-alkyl, C₂-C₈-alkenyl, C₂-C₈-alkynyl or C₃-C₆-cycloalkyl, where the radicals alkyl, alkenyl and alkynyl may be partially or fully halogenated and may carry one to three groups R^{v}, into the esters of the formula V, which are then, using amines R^{a}-X-NH₂ and added dehydrating agents, converted into the amides IA' and further, in the presence of carbon tetrahalide and triarylphosphine, into the imine halides of the formula VI and finally, with alcohols of the formula R^{b}OH and bases, into the imino ethers of the formula IC.

8. An ester of the formula V 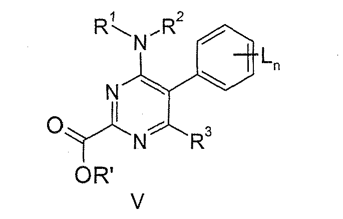 in which the substituents R¹, R², R³ and Lₙ are as defined in claim 1 and R' is C₁-C₈-alkyl, C₂-C₈-alkenyl, C₂-C₈-alkynyl or C₃-C₆-cycloalkyl, where the radicals alkyl, alkenyl and alkynyl may be partially or fully halogenated and may carry one to three groups R^{v}.

9. The ester according to claim 8 where R' is isopropyl.

10. An imine halide of the formula VI 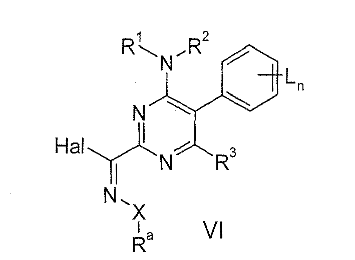 where the substituents Lₙ, R¹, R², R³, X and R^{a} are as defined in claim 1 and Hal is fluorine, chlorine, bromine or iodine.

11. A pesticide which comprises a solid or liquid carrier and a compound of the formula I according to claim 1.

12. A pesticide which comprises a solid or liquid carrier and a compound of the formula V according to either of claims 8 and 9.

13. A method for controlling phytopathogenic harmful fungi, which comprises treating the fungi or the materials, plants, the soil or the seeds to be protected against fungal attack with an effective amount of a compound of the formula I according to claim 1.

14. A method for controlling phytopathogenic harmful fungi, which comprises treating the fungi or the materials, plants, the soil or the seeds to be protected against fungal attack with an effective amount of a compound of the formula V according to either of claims 8 and 9.

## Revendications

1. Pyrimidines 2-substituées de la formule I : 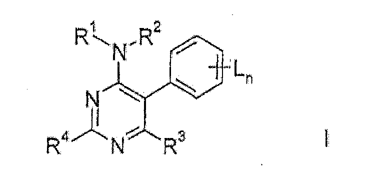 dans laquelle l'indice et les substituants ont la signification suivante :
n est un nombre entier de 1 à 5,
L représente de l'halogène ou un groupe cyano, cyanato (OCN) , alkyle en C₁-C₈, alcényle en C₂-C₈, alcynyle en C₂-C₈, alcoxy en C₁-C₆, alcényloxy en C₂-C₈, alcynyloxy en C₂-C₈, cycloalkyle en C₃-C₆, cycloalcényle en C₄-C₆, cycloalkyloxy en C₃-C₆, cycloalcényloxy en C₄-C₆, nitro, -C(=O)-A, -C(=O)-O-A, -C(=O) -N(A')A, C(A')(=N-OA), N(A')A, N(A')-C(=O)-A, N(A")-C(=O)-N(A')A, S(=O)ₘ-A, S(=O)ₘ-O-A ou S(=O)ₘ-N(A')A,
m valant 0, 1 ou 2,
A, A', A'' représentant, indépendamment l'un de l'autre, de l'hydrogène ou un groupe alkyle en C₁-C₆, alcényle en C₂-C₆, alcynyle en C₂-C₆, cycloalkyle en C₃-C₈, cycloalcényle en C₃-C₈, phényle, les radicaux organiques pouvant être partiellement ou totalement halogénés ou substitués par un groupe nitro, cyanato, cyano ou alcoxy en C₁-C₄, A et A' pouvant représenter, conjointement avec les atomes sur lesquels ils sont fixés, un hétérocycle pentagonal à hexagonal saturé, partiellement insaturé ou aromatique, contenant un à quatre hétéroatomes du groupe de O, N ou S,
les groupes aliphatiques des définitions de radicaux de L pouvant à leur tour être partiellement ou totalement halogénés ou porter un à quatre groupes R^{u} :
R^{u} représentant un groupe cyano, alcoxy en C₁-C₆, cycloalkyle en C₃-C₆, alcényloxy en C₂-C₈, alcynyloxy en C₂-C₈, cycloalcényle en C₄-C₆, cycloalkyloxy en C₃-C₆, cycloalcényloxy en C₄-C₆, -C(=O)-A, -C(=O)-O-A, -C(=O)-N(A')A, C(A')(=N-OA), N(A')A, N(A')-C (=O)-A, N(A")-C(=O)-N(A')A, S(=O)ₘ-A, S(=O)ₘ-O-A ou S(=O)ₘ-N(A')A,
R¹, R² représentent, indépendamment l'un de l'autre, un groupe alkyle en C₁-C₆, alcényle en C₂-C₆, alcynyle en C₂-C₆, cycloalkyle en C₃-C₆, halogénocycloalkyle en C₃-C₆, les groupes aliphatiques des définitions de radicaux de R¹ et R² pouvant à leur tour être partiellement ou totalement halogénés ou porter un à quatre groupes R^{v} :
R^{v} représentant un groupe cyano, cycloalkyle en C₃-C₆, cycloalcényle en C₄-C₆, hydroxy, alcoxy en C₁-C₆, alcényloxy en C₂-C₈, alcynyloxy en C₂-C₈, cycloalkyloxy en C₃-C₆, cycloalcényloxy en C₄-C₆, alkylthio en C₁-C₆, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A ou S(=O)ₘ-N(A')_{A} ou phényle, la partie phényle pouvant porter un a trois radicaux choisis parmi le groupe des halogènes et des groupes alkyle en C₁-C₆, alcényle en C₂-C₆, alcynyle en C₂-C₆, cycloalkyle en C₃-C₆, halogénoalkyle en C₁-C₆, alcoxy en C₁-C₆, cyano, nitro, -C(=O)-A, -C(=O)-O-A,-C(=O)-N(A')A, C(A')(=N-OA), N(A')A,
R² pouvant représenter en supplément de l'hydrogène,
R¹ et R² pouvant aussi former, conjointement à l'atome d'azote sur lequel ils sont fixés, un noyau pentagonal ou hexagonal saturé ou insaturé qui peut être interrompu par un groupe éther-(-O-), carbonyle-(C=_{O})-, thio-(-S-), sulfoxyle-(-S[=O]-) ou sulfényle-(-SO₂-) ou un autre amino-(N (R^{a}))-, où R^{a} représente de l'hydrogène ou un groupe alkyle en C₁-C₆, et/ou pourant contenir un ou plusieurs substituants du groupe des halogènes et des groupes alkyle en C₁-C₆, halogénoalkyle en C₁-C₆ ou oxy-C₁-C₃-alkylénoxy,
R³ représente un halogène ou un groupe cyano, alkyle en C₁-C₄, alcényle en C₂-C₄, alcynyle en C₂-C₄, cycloalkyle en C₃-C₆, alcoxy en C₁-C₄, alcényloxy en C₃-C₄, alcynyloxy en C₃-C₄, alkylthio en C₁-C₆, di-(C₁-C₆-alkyl)amino ou alkylamino en C₁-C₆, les radicaux alkyle, alcényle et alcynyle de R³ pouvant être substitués par de l'halogène ou un groupe cyano, nitro, alcoxy en C₁-C₂ ou alcoxycarbonyle en C₁-C₄,
R⁴ répond à une des formules : 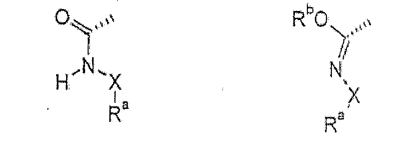 dans lesquelles
X représente une liaison directe, -(C=O)-,-(C=O)-NH-, -(C=O)-O-, -O-, -NR^{c}-, -CH₂O-(C=O)-, -C=C-(C=O)-, où la partie de
molécule respectivement à gauche est fixée sur l'atome d'azote,
R^{a} représente de l'hydrogène ou un groupe alkyle en C₁-C₆, alcényle en C₂-C₈, alcynyle en C₂-C₈ ou benzyle,
R^{b} représente de l'hydrogène ou un groupe alkyle en C₁-C₆, alcényle en C₂-C₆ ou alcynyle en C₂-C₈,
R^{c} représente de l'hydrogène ou un groupe alkyle en C₁-C₆, alcényle en C₂-C₆, alcynyle en C₂-C₈, benzyle ou acyle en C₁-C₆,
les groupes aliphatiques, alicycliques ou aromatiques des définitions de radicaux de R^{a}, R^{b} et/ou R^{c} pouvant à leur tour porter un à quatre groupes R^{w} :
R^{w} représentant de l'halogène ou un groupe cyano, OR^{x}, NHR^{x}, SR^{x}, alkyle en C₁-C₆, alcényle en C₂-C₆, alcynyle en C₂-C₆, alcoxy en C₁-C₆, alcoxycarbonyle en C₁-C₄, acylamino en C₁-C₄, [1,3]dioxolane-C₁-C₄-alkyle, [1,3]-dioxane-C₁-C₄-alkyle, où
R^{x} représente de l'hydrogène ou un groupe alkyle en C₁-C₆, alcényle en C₂-C₈, alcynyle en C₂-C₈ ou benzyle.

2. Pyrimidines 2-substituées de la formule I suivant la revendication 1, dans lesquelles l'indice et les substituants ont la signification suivante :
n est un nombre entier de 1 à 3, au moins un substituant L étant en position ortho sur le noyau de phényle,
L représente de l'halogène ou un groupe cyano, méthyle, méthoxy, -C(=O)-O-A, -C(=O)-N(A')A, C(A')(=N-OA), N(A')A, N(A')-C(=O)-A,
A, A' représentant, indépendamment l'un de l'autre, de l'hydrogène ou un groupe alkyle en C₁-C₆, alcényle en C₂-C₆, alcynyle en C₂-C₆, phényle, les radicaux organiques pouvant être halogénés partiellement ou totalement ou substitués par un groupe alcoxy en C₁-C₄, A et A' pouvant former, conjointement aux atomes sur lesquels ils sont fixés, un hétérocycle saturé pentagonal à hexagonal contenant un ou deux hétéroatomes du groupe O, N ou S,
les groupes aliphatiques des définitions de radicaux de L pouvant à leur tour être partiellement ou totalement halogénés,
R¹ R² représentent, indépendamment l'un de l'autre, un groupe alkyle en C₁-C₆, alcényle en C₂-C₆, alcynyle en C₂-C₆, halogénoalkyle en C₁-C₆, halogénoalcényle en C₂-C₆ ou halogénoalcynyle en C₂-C₆,
R² pouvant représenter en supplément de l'hydrogène,
R¹ et R² pouvant aussi former, conjointement à l'atome d'azote sur lequel ils sont fixés, un noyau pentagonal ou hexagonal saturé ou insaturé qui peut être interrompu par un groupe éther-(-O-) ou un autre amino-N-(R^{a})-, R^{a} représentant de l'hydrogène ou un groupe alkyle en C₁-C₆, et/ou pouvant contenir un ou plusieurs substituants du groupe des halogènes et des groupes alkyle en C₁-C₆, halogénoalkyle en C₁-C₆ et oxy-C₁-C₃-alkylénoxy,
R³ représente de l'halogène ou un groupe cyano, alkyle en C₁-C₄, alcoxy en C₁-C₄ ou halogénoalkyle en C₁-C₄,
R⁴ correspond à une des formules : 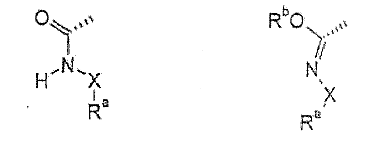 dans lesquelles
X représente une liaison directe, -(C=O)-,-(C=O)-NH-, - (C=O)-O-, -O-, -NR^{c}-, où la partie de molécule respectivement à gauche est fixée sur l'atome d'azote,
R^{a} représente de l'hydrogène ou un groupe méthyle, allyle ou propargyle,
R^{b} représente de l'hydrogène ou un groupe alkyle en C₁-C₄, allyle ou propargyle,
R^{c} représente de l'hydrogène ou un groupe méthyle ou acyle en C₁-C₄,
les groupes aliphatiques des définitions de radicaux de R^{a}, R^{b} et/ou R^{c} pouvant à leur tour porter un ou deux groupes R^{w} :
R^{w} représentant de l'halogène ou un groupe OR^{x}, NHR^{x}, alkyle en C₁-C₆, alcoxycarbonyle en C₁-C₄, acylamino en C₁-C₄, [1, 3]dioxolane-C₁-C₄-alkyle, [1, 3] dioxane-C₁-C₄-alkyle, où R^{x} représente de l'hydrogène ou un groupe méthyle, allyle ou propargyle.

3. Pyrimidines 2-substituées suivant la revendication 1, dans lesquelles R³ représente du chlore ou un groupe cyano, méthyle ou méthoxy.

4. Pyrimidines 2-substituées suivant la revendication 1, dans lesquelles R⁴ répond à une formule : 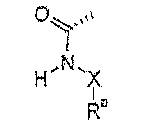 dans laquelle X représente une liaison directe -O- ou -(C=O)-O- et R^{a} représente de l'hydrogène ou un groupe alkyle en C₁-C₆.

5. Pyrimidines 2-substituées suivant l'une des revendications 1 à 4, dans lesquelles le groupe phényle substitué par Lₙ représente le groupe B : 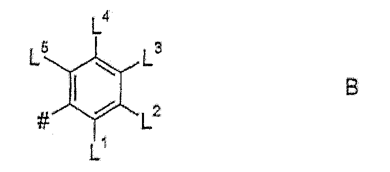 dans laquelle # est l'emplacement de liaison avec le squelette de pyrimidine, et
L¹ représente du fluor, du chlore, CH₃ ou CF₃,
L², L⁴ représentent, indépendamment l'un de l'autre, de l'hydrogène, CH₃ ou du fluor,
L³ représente de l'hydrogène, du fluor, du chlore, ou un groupe cyano, CH₃, SCH₃, OCH₃, SO₂CH₃, NH-C(=O)CH₃, N(CH₃)-C(=O)CH₃ ou COOCH₃ et
L⁵ représente de l'hydrogène, du fluor, du chlore ou CH₃.

6. Procédé de préparation des composés IA, par hydrolyse des nitriles de la formule IV : 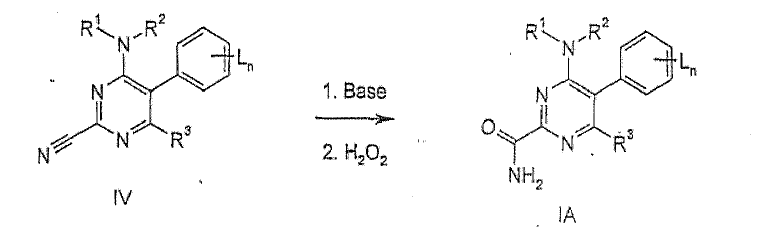 où les substituants R¹, R², R³ et L ainsi que l'indice n ont la signification donnée dans la revendication 1, **caractérisé en ce qu'**on hydrolyse en présence d'une base et de peroxyde d'hydrogène.

7. Procédé de préparation des composés suivant l'invention IA' et IC, où les substituants Lₙ, R¹, R², R³, X, R^{a} et R^{b} ont la signification donnée dans la revendication 1, en partant de nitriles de la formule IV : 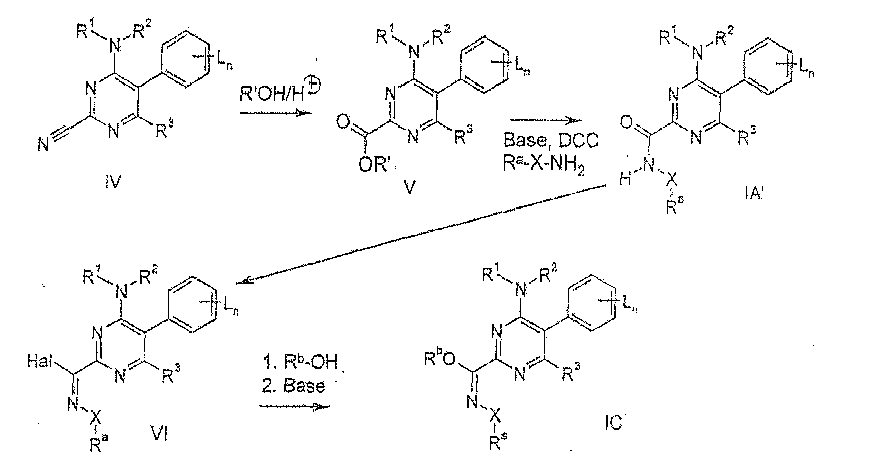 qui sont mis à réagir avec des alcools de la formule R'OH, où R' représente un groupe alkyle en C₁-C₈, alcényle en C₂-C₈, alcynyle en C₂-C₈ ou cycloalkyle en C₃-C₆, les radicaux alkyle, alcényle et alcynyle pouvant être partiellement ou totalement halogénés et porter un à trois groupes R^{v}, pour former les esters de la formule V, puis avec des amines R^{a}-X-NH₂ avec addition d'agents déshydratants pour former les amides IA' et en outre, en présence de tétrahalogénure de carbone et de triarylphosphine, pour former les halogénures d'imine de la formule VI et enfin avec des alcools de la formule R^{b}OH et des bases pour former les iminoéthers de la formule IC.

8. Esters de la formule V : 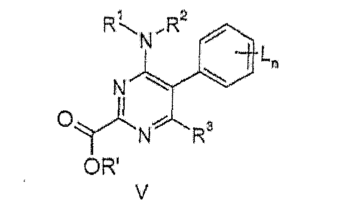 dans laquelle les substituants R¹, R², R³ et Lₙ ont la signification donnée dans la revendication 1 et R' représente un groupe alkyle en C₁-C₈, alcényle en C₂-C₈, alcynyle en C₂-C₈ ou cycloalkyle en C₃-C₆, les radicaux alkyle, alcényle et alcynyle pouvant être partiellement ou totalement halogénés et porter un à trois groupes R^{v}.

9. Esters V suivant la revendication 8, où R' représente un groupe isopropyle.

10. Halogénures d'imine de la formule VI : 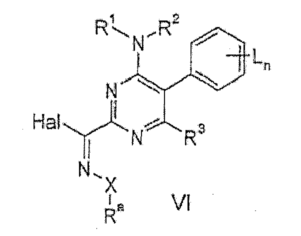 dans laquelle les substituants Lₙ, R¹, R², R³, X et R³, X et R² ont la signification donnée dans la revendication 1 et Hal représente du fluor, du chlore, du brome ou de l'iode.

11. Produit pesticide, contenant un support solide ou liquide et un composé de la formule I suivant la revendication 1.

12. Produit pesticide contenant un support solide ou liquide et un composé de la formule V suivant l'une des revendications 8 et 9.

13. Procédé de lutte contre les champignons nuisibles phytopathogènes, **caractérisé en ce qu'**on traite les champignons ou les matériels, plantes, sols ou semences à protéger d'une infestation par les champignons, avec une quantité efficace d'un composé de la formule I suivant la revendication 1.

14. Procédé de lutte contre des champignons nuisibles phytopathogènes, **caractérisé en ce qu'**on traite les champignons ou les matériels, plantes, sols ou semences à protéger d'une infestation par les champignons, avec une quantité efficace d'un composé de la formule V suivant l'une des revendications 8 et 9.
